# EUROPÄISCHE PATENTSCHRIFT

(11) **EP 4 143 779 B1**
(45) Veröffentlichungstag und Bekanntmachung des Hinweises auf die Patenterteilung: **04.06.2025**
(21) Anmeldenummer: 21820574.8
(22) Anmeldetag: 29.11.2021
(51) Int. Cl.: G06T 5/60, G06T 5/92, G06T 5/10

(54) **MASCHINELLES LERNEN IM BEREICH DER KONTRASTMITTELVERSTÄRKTEN RADIOLOGIE**
MACHINE LEARNING IN THE FIELD OF RADIOLOGY WITH CONTRAST AGENT
APPRENTISSAGE AUTOMATIQUE DANS LE DOMAINE DE LA RADIOLOGIE ASSISTÉE PAR CONTRASTE

(30) Priorität: 02.03.2021 EP 21160325; 07.04.2021 EP 21167116
(43) Veröffentlichungstag der Anmeldung: 08.03.2023
(73) Patentinhaber: Bayer Aktiengesellschaft, 51373 Leverkusen (DE)
(72) Erfinder: LENGA, Matthias, 51373 Leverkusen (DE); PURTORAB, Marvin, 30655 Hannover (DE)
(74) Vertreter: BIP Patents
(86) Internationale Anmeldenummer: PCT/EP2021/083321
(87) Internationale Veröffentlichungsnummer: WO 2022/184297

(56) Entgegenhaltungen:
- WO-A1-2019/074938
- SONG HK ET AL: "k-space weighted image contrast (KWIC) for contrast manipulation in projection reconstruction MRI", MAGNETIC RESONANCE IN MEDICINE,, vol. 44, no. 6, 1 December 2000 (2000-12-01), pages 825 - 832, XP002333653, ISSN: 0740-3194, DOI: 10.1002/1522-2594(200012)44:6<825::AID-MRM2>3.0.CO;2-D

## Beschreibung

Die vorliegende Erfindung befasst sich mit dem technischen Gebiet der Erzeugung von künstlichen kontrastmittel verstärkten radiologischen Aufnahmen mittels Methoden des maschinellen Lernens.

WO2019/074938A1 offenbart ein Verfahren zur Reduzierung der Menge an Kontrastmittel bei der Erzeugung von radiologischen Aufnahmen mit Hilfe eines künstlichen neuronalen Netzes.

In einem ersten Schritt wird ein Trainingsdatensatz erzeugt. Der Trainingsdatensatz umfasst für eine Vielzahl an Personen für jede Person i) eine native radiologische Aufnahme (*zero-contrast image*), ii) eine radiologische Aufnahme nach der Applikation einer geringen Menge an Kontrastmittel (*lowcontrast image*) und iii) eine radiologische Aufnahme nach der Applikation einer Standardmenge an Kontrastmittel (*full-contrast image*). Der Begriff "Vielzahl" bedeutet vorzugsweise mehr als 10, noch mehr bevorzugt mehr als 100.

In einem zweiten Schritt wird ein künstliches neuronales Netz trainiert, für jede Person des Trainingsdatensatzes auf Basis der nativen Aufnahme und der Aufnahme nach Applikation einer geringen Menge an Kontrastmittel eine künstliche radiologische Aufnahme vorherzusagen, die einen Aufnahmebereich nach der Applikation der Standardmenge an Kontrastmittel zeigt. Die gemessene radiologische Aufnahme nach der Applikation einer Standardmenge an Kontrastmittel dient beim Training jeweils als Referenz (*ground truth*).

In einem dritten Schritt kann das trainierte künstliche neuronale Netz verwendet werden, für eine neue Person auf Basis einer nativen Aufnahme und einer radiologischen Aufnahme nach der Applikation einer geringen Menge an Kontrastmittel eine künstliche radiologische Aufnahme vorherzusagen, die den aufgenommenen Bereich so zeigt wie er aussehen würde, wenn eine Standardmenge an Kontrastmittel appliziert worden wäre.

Das in WO2019/074938A1 offenbarte Verfahren weist Nachteile auf.

So ist eine Co-Registrierung der radiologischen Aufnahmen erforderlich, um die einzelnen radiologischen Aufnahmen so anzupassen, dass die Pixel/Voxel miteinander übereinstimmen, d.h. dass ein Pixel/Voxel einer radiologischen Aufnahme einer Person denselben Untersuchungsbereich zeigt wie das Pixel/Voxel einer anderen radiologischen Aufnahme der Person. Stimmen die radiologischen Aufnahmen nicht überein, so treten in den künstlich erzeugten radiologischen Aufnahmen Artefakte auf, die kleine anatomische Strukturen in dem Aufnahmebereich überdecken und/oder verfälschen und/oder vortäuschen können.

Ferner werden in dem in WO2019/074938A1 offenbarten Verfahren zur Vorhersage stets komplette radiologische Aufnahmen verwendet. Insbesondere dann, wenn beim Trainieren des künstlichen neuronalen Netzes und der Vorhersage einer künstlichen radiologischen Aufnahme nicht nur die oben unter den Ziffern i) und ii) genannten radiologischen Aufnahmen verwendet werden, sondern weitere radiologische Aufnahmen - z.B. radiologische Aufnahme nach Applikation variierender Mengen an Kontrastmittel - kann der Rechenaufwand zur Erzeugung der künstlichen radiologischen Aufnahme schnell sehr groß werden. Es ist denkbar, dass viel Zeit zur Berechnung künstlicher radiologischer Aufnahmen beansprucht wird und/oder spezielle und/oder teure Hardware nötig ist, um die Berechnungen (in einer angemessenen Zeitspanne) durchführen zu können. Es ist denkbar, dass radiologische Aufnahmen in Teilbereiche (*patches*) zerlegt werden (müssen), und diese Teilbereiche separat voneinander verarbeitet werden (müssen), um den Arbeitsspeicher des Computers nicht mit allzu großen radiologischen Aufnahmen zu überlasten. Eine solche Vorgehensweise kann jedoch zu Artefakten an den Nahtstellen führen, wenn die separat voneinander prozessierten Teilbereiche wieder zu einer kompletten radiologischen Aufnahme zusammengefügt werden (*Stitching*-Artefakte). Die nachträgliche Entfernung solcher *Stitching*-Artefakte bedeutet einen zusätzlichen Aufwand und die Gefahr von Fehlern in den synthetisch erzeugten radiologischen Aufnahmen, die von einem Radiologen fehlinterpretiert werden könnten (Gefahr der Fehldiagnose).

Ausgehend vom beschriebenen Stand der Technik stellt sich daher die technische Aufgabe, eine Lösung zur Erzeugung von künstlichen radiologischen Aufnahmen zu erzeugen, die tolerant gegenüber Fehlern bei der Co-Registrierung ist und/oder bei der die Vorhersage weniger Rechenleistung erfordert und/oder bei der der Rechenaufwand an die gegebene Hardware und/oder die zur Verfügung stehende Zeit angepasst werden kann und/oder bei der die Gefahr von Artefakten (insbesondere *Stitching*-Artefakten) vermindert werden kann.

Diese Aufgabe wird durch die Gegenstände der unabhängigen Patentansprüche gelöst. Bevorzugte Ausführungsformen der vorliegenden Erfindung finden sich in den abhängigen Patentansprüchen, in der vorliegenden Beschreibung und in den Zeichnungen.

Ein erster Gegenstand der vorliegenden Erfindung ist ein computer-implementiertes Verfahren umfassend die Schritte:
- Empfangen einer ersten Repräsentation eines Untersuchungsbereich eines Untersuchungsobjekts im Frequenzraum, wobei die erste Repräsentation den Untersuchungsbereich ohne Kontrastmittel oder nach Applikation einer ersten Menge an Kontrastmittel repräsentiert,
- Empfangen einer zweiten Repräsentation des Untersuchungsbereichs des Untersuchungsobjekts im Frequenzraum, wobei die zweite Repräsentation den Untersuchungsbereich nach einer Applikation einer zweiten Menge an Kontrastmittel repräsentiert,
- Zuführen zumindest eines Teils der ersten Repräsentation und zumindest eines Teils der zweiten Repräsentation einem Modell des maschinellen Lernens,
- Empfangen, von dem Modell des maschinellen Lernens, einer dritten Repräsentation des Untersuchungsbereichs im Frequenzraum, wobei die dritte Repräsentation den Untersuchungsbereich nach Applikation einer dritten Menge an Kontrastmittel repräsentiert,
- Erzeugen einer Repräsentation des Untersuchungsbereichs in einer Ortsraumdarstellung auf Basis der dritten Repräsentation,
- Ausgeben und/oder Speichern der Repräsentation des Untersuchungsbereichs in der Ortsraumdarstellung.

Ein weiterer Gegenstand der vorliegenden Erfindung ist ein Computersystem umfassend
- eine Empfangseinheit,
- eine Steuer- und Recheneinheit und
- eine Ausgabeeinheit,

- wobei die Steuer- und Recheneinheit konfiguriert ist, die Empfangseinheit zu veranlassen, mindestens zwei Repräsentationen eines Untersuchungsbereichs eines Untersuchungsobjekts zu empfangen, eine erste Repräsentation und eine zweite Repräsentation, wobei die erste Repräsentation den Untersuchungsbereich im Frequenzraum ohne Kontrastmittel oder nach Applikation einer ersten Menge an Kontrastmittels repräsentiert, wobei die zweite Repräsentation den Untersuchungsbereich im Frequenzraum nach einer Applikation einer zweiten Menge an Kontrastmittel repräsentiert,
- wobei die Steuer- und Recheneinheit konfiguriert ist, zumindest einen Teil der ersten Repräsentation und zumindest einen Teil der zweiten Repräsentation einem Modell des maschinellen Lernens zuzuführen,
- wobei die Steuer- und Recheneinheit konfiguriert ist, von dem Modell des maschinellen Lernens eine dritte Repräsentation des Untersuchungsbereichs zu empfangen, wobei die dritte Repräsentation den Untersuchungsbereich im Frequenzraum nach Applikation einer dritten Menge an Kontrastmittel repräsentiert,
- wobei die Steuer- und Recheneinheit konfiguriert ist, auf Basis der dritten Repräsentation eine Repräsentation des Untersuchungsbereichs im Ortsraum zu erzeugen,
- wobei die Steuer- und Recheneinheit konfiguriert ist, die Ausgabeeinheit zu veranlassen, die Repräsentation des Untersuchungsbereichs im Ortsraum auszugeben und/oder zu speichern.

Ein weiterer Gegenstand der vorliegenden Erfindung ist ein Computerprogramm, das in einen Arbeitsspeicher eines Computersystems geladen werden kann und dort das Computersystem dazu veranlasst, folgende Schritte ausführen:
- Empfangen einer ersten Repräsentation eines Untersuchungsbereich eines Untersuchungsobjekts im Frequenzraum, wobei die erste Repräsentation den Untersuchungsbereich ohne Kontrastmittel oder nach Applikation einer ersten Menge an Kontrastmittel repräsentiert,
- Empfangen einer zweiten Repräsentation des Untersuchungsbereichs des Untersuchungsobjekts im Frequenzraum, wobei die zweite Repräsentation den Untersuchungsbereich nach einer Applikation einer zweiten Menge an Kontrastmittel repräsentiert,
- Zuführen zumindest eines Teils der ersten Repräsentation und zumindest eines Teils der zweiten Repräsentation einem Modell des maschinellen Lernens,
- Empfangen, von dem Modell des maschinellen Lernens, einer dritten Repräsentation des Untersuchungsbereichs im Frequenzraum, wobei die dritte Repräsentation den Untersuchungsbereich nach Applikation einer dritten Menge an Kontrastmittel repräsentiert,
- Erzeugen einer Repräsentation des Untersuchungsbereichs in einer Ortsraumdarstellung auf Basis der dritten Repräsentation,
- Ausgeben und/oder Speichern der Repräsentation des Untersuchungsbereichs in der Ortsraumdarstellung.

Die Erfindung wird nachstehend näher erläutert, ohne zwischen den Erfindungsgegenständen (Verfahren, Computersystem, Computerprogramm) zu unterscheiden. Die nachfolgenden Erläuterungen sollen vielmehr für alle Erfindungsgegenstände in analoger Weise gelten, unabhängig davon, in welchem Kontext (Verfahren, Computersystem, Computerprogramm) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Mit Hilfe der vorliegenden Erfindung kann eine künstliche radiologische Aufnahme eines Untersuchungsbereichs eines Untersuchungsobjekts erzeugt werden.

Das "Untersuchungsobjekt" ist üblicherweise ein Lebewesen, vorzugsweise ein Säugetier, ganz besonders bevorzugt ein Mensch.

Der "Untersuchungsbereich" ist ein Teil des Untersuchungsobjekts, zum Beispiel ein Organ oder ein Teil eines Organs.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

Der Untersuchungsbereich wird einer radiologischen Untersuchung unterzogen.

Die "Radiologie" ist das Teilgebiet der Medizin, das sich mit der Anwendung elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlung wie Gammastrahlung oder Elektronen zum Einsatz. Da ein wesentlicher Einsatzzweck die Bildgebung ist, werden auch andere bildgebende Verfahren wie die Sonografie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Erfindung umfasst damit insbesondere die folgenden Untersuchungsmethoden: Computertomografie, Magnetresonanztomografie, Sonografie.

In einer bevorzugten Ausführungsform der vorliegenden Erfindung handelt es sich bei der radiologischen Untersuchung um eine magnetresonanztomographische Untersuchung.

Die Magnetresonanztomographie, abgekürzt MRT oder MR (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten MR-Daten liegen zunächst als Rohdaten in einem Frequenzraum vor, und können durch anschließende inverse Fourier-Transformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt. Dabei beschreibt die T1-Relaxation den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxationszeit oder Spin-Gitter-Relaxationszeit genannt. Die T2-Relaxation beschreibt in analoger Weise den Übergang der Transversalmagnetisierung in ihren Gleichgewichtszustand.

Bei radiologischen Untersuchungen werden häufig Kontrastmittel zur Kontrastverstärkung eingesetzt. "Kontrastmittel" sind Substanzen oder Gemische von Substanzen, die die Darstellung von Strukturen und Funktionen des Körpers bei bildgebenden Verfahren wie Röntgendiagnostik, Magnetresonanztomografie und Sonografie verbessern.

In der Computertomographie werden meist iodhaltige Lösungen als Kontrastmittel eingesetzt. In der Magnetresonanztomographie (MRT) werden üblicherweise superparamagnetische Substanzen (z.B. Eisenoxidnanopartikel, superparamagnetische Eisen-Platin-Partikel (SIPPs)) oder paramagnetische Substanzen (z.B. Gadolinium-Chelate, Mangan-Chelate) als Kontrastmittel verwendet. Im Fall der Sonografie werden üblicherweise Flüssigkeiten, die gasgefüllte Mikrobläschen (*microbubbles*) enthalten, intravenös verabreicht. Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wpcontent/uploads/2017/10/contrast-agents-tutorial.pdf, M. R. Nough et al.: Radiographie and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review of Risk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide*). Beispiele für paramagnetische Kontrastmittel sind Gadolinium-Chelate wie Gadopentetat-Dimeglumin (Handelsname: Magnevist^{®} u.a.), Gadotersäure (Dotarem^{®}, Dotagita^{®}, Cyclolux^{®}), Gadodiamid (Omniscan^{®}), Gadoteridol (ProHance^{®}) und Gadobutrol (Gadovist^{®}).

Mit Hilfe der vorliegenden Erfindung kann eine künstliche radiologische Aufnahme erzeugt werden, die den Untersuchungsbereich eines Untersuchungsobjekts so zeigt, wie er aussehen würde, wenn dem Untersuchungsobjekt/Untersuchungsbereich eine spezifische Menge an Kontrastmittel verabreicht worden wäre, ohne dass diese spezifische Menge tatsächlich verabreicht werden muss.

Dazu wird ein Modell des maschinellen Lernens (in dieser Beschreibung auch als Vorhersagemodell bezeichnet) eingesetzt, das anhand von Trainingsdaten lernt, wie sich unterschiedliche Mengen an Kontrastmittel auf den Kontrast einer radiologischen Aufnahme eines Untersuchungsbereichs auswirken. Das trainierte Modell kann dann genutzt werden, um eine radiologische Aufnahme mit einer Kontrastverstärkung vorherzusagen, die sich nach Applikation einer spezifischen Menge an Kontrastmittel ergeben würde, ohne dass diese Menge tatsächlich appliziert werden muss.

Die vorliegende Erfindung kann also beispielsweise, ähnlich wie in WO2019/074938A1 beschrieben, zur Reduktion der Menge an appliziertem Kontrastmittel eingesetzt werden, ohne auf die Vorteile einer hohen Kontrastmittelgabe (d.h. eine hohe Kontrastverstärkung) verzichten zu müssen.

Die Vorhersage von radiologischen Aufnahmen erfolgt jedoch nicht auf Basis von Bildern (*images*), wie dies in WO2019/074938A1 beschrieben ist. Die in WO2019/074938A1 zur Vorhersage von künstlichen radiologischen Aufnahmen verwendeten Bilder (*images*) sind Repräsentationen (Darstellungen) eines Untersuchungsbereichs im Ortsraum (auch *"image space"* genannt).

Erfindungsgemäß wird das Modell des maschinellen Lernens mit Hilfe von Repräsentationen eines Untersuchungsbereichs einer Vielzahl an Untersuchungsobjekten im Frequenzraum trainiert und auch die Vorhersage erfolgt auf Basis von Repräsentationen des Untersuchungsbereichs im Frequenzraum (auch als Ortsfrequenzraum oder Fourier-Raum oder Frequenzdomäne oder Fourier-Darstellung bezeichnet).

In der Magnetresonanztomografie fallen die Rohdaten aufgrund des oben beschriebenen Messverfahrens üblicherweise als so genannte k-Raum-Daten an. Bei diesen k-Raum-Daten handelt es sich um eine Darstellung eines Untersuchungsbereichs in einem Frequenzraum. Solche k-Raum-Daten können zum Trainieren eines Modells des maschinellen Lernens, zum Validieren des Modells und zur Vorhersage mit Hilfe des trainierten Modells gemäß der vorliegenden Erfindung verwendet werden.

Liegen hingegen Repräsentationen im Ortsraum vor, so können diese beispielsweise mittels Fourier-Transformation in eine Repräsentation im Frequenzraum umgewandelt (transformiert) werden.

Liegt also eine radiologische Aufnahme eines Untersuchungsbereichs in Form eines zwei- oder dreidimensionalen Bildes im Ortsraum vor, so kann diese Repräsentation des Untersuchungsbereichs beispielsweise durch eine 2D-oder 3D-Fourier-Transformation in eine zwei- bzw. dreidimensionale Repräsentation des Untersuchungsbereichs im Frequenzraum überführt werden.

Umgekehrt können Repräsentationen im Frequenzraum durch inverse Fourier-Transformation in eine Repräsentation im Ortsraum umgewandelt (transformiert) werden.

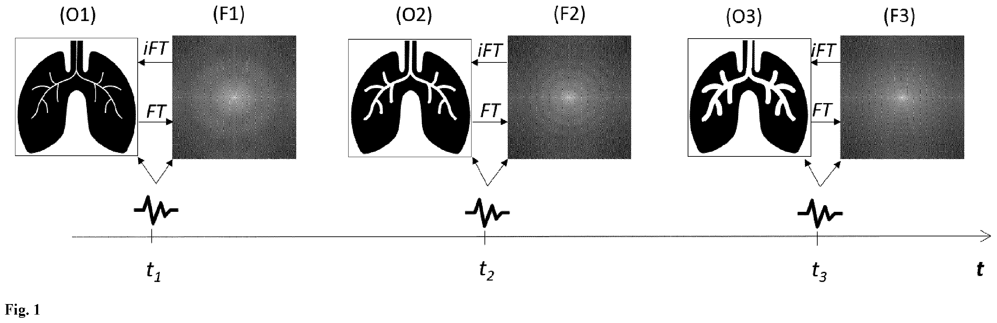

Fig. 1 zeigt schematisch und beispielhaft den Zusammenhang zwischen Repräsentationen eines Untersuchungsbereichs im Ortsraum und im Frequenzraum. In Fig. 1 ist ein Zeitstrahl dargestellt. Zu drei verschiedenen Zeitpunkten *t₁, t*₂ und *t*₃ werden messtechnisch Repräsentationen eines Untersuchungsbereichs erzeugt. Bei dem Untersuchungsbereich handelt es sich um die Lunge eines Menschen. Zum Zeitpunkt *t*₁ wird eine erste Repräsentation erzeugt. Dabei kann es sich um eine Repräsentation O1 des Untersuchungsbereichs (Lunge) im Ortsraum oder um eine Repräsentation F 1 des Untersuchungsbereichs (Lunge) im Frequenzraum handelt. Die Repräsentation O1 des Untersuchungsbereichs im Ortsraum lässt sich mittels Fourier-Transformation (*FT*) in eine Repräsentation F1 des Untersuchungsbereichs im Frequenzraum umwandeln. Die Repräsentation F1 des Untersuchungsbereichs im Frequenzraum lässt sich mittels inverser Fourier-Transformation (*iFT*) in eine Repräsentation O1 des Untersuchungsbereichs im Ortsraum umwandeln.

Bei den Repräsentationen O1 und F1 kann es sich z.B. um Repräsentationen des Untersuchungsbereichs ohne ein Kontrastmittel oder nach Applikation einer ersten Menge an Kontrastmittel handeln.

Die beiden Repräsentation O1 und F1 umfassen die gleichen Informationen über den Untersuchungsbereich, nur in einer anderen Darstellung.

Zum Zeitpunkt *t*₂ wird eine weitere Repräsentation erzeugt. Dabei kann es sich um eine Repräsentation O2 des Untersuchungsbereichs (Lunge) im Ortsraum oder um eine Repräsentation F2 des Untersuchungsbereichs (Lunge) im Frequenzraum handelt. Die Repräsentation O2 des Untersuchungsbereichs im Ortsraum lässt sich mittels Fourier-Transformation (*FT*) in eine Repräsentation F2 des Untersuchungsbereichs im Frequenzraum umwandeln. Die Repräsentation F2 des Untersuchungsbereichs im Frequenzraum lässt sich mittels inverser Fourier-Transformation (*iFT*) in eine Repräsentation O2 des Untersuchungsbereichs im Ortsraum umwandeln.

Bei den Repräsentationen O2 und F2 kann es sich z.B. um Repräsentationen des Untersuchungsbereichs nach Applikation einer zweiten Menge an Kontrastmittel handeln.

Die beiden Repräsentation O2 und F2 umfassen die gleichen Informationen über den Untersuchungsbereich, nur in einer anderen Darstellung.

Zum Zeitpunkt *t*₃ wird eine weitere Repräsentation erzeugt. Dabei kann es sich um eine Repräsentation O3 des Untersuchungsbereichs (Lunge) im Ortsraum oder um eine Repräsentation F3 des Untersuchungsbereichs (Lunge) im Frequenzraum handelt. Die Repräsentation O3 des Untersuchungsbereichs im Ortsraum lässt sich mittels Fourier-Transformation (*FT*) in eine Repräsentation F3 des Untersuchungsbereichs im Frequenzraum umwandeln. Die Repräsentation F3 des Untersuchungsbereichs im Frequenzraum lässt sich mittels inverser Fourier-Transformation (*iFT*) in eine Repräsentation O3 des Untersuchungsbereichs im Ortsraum umwandeln.

Bei den Repräsentationen O3 und F3 kann es sich z.B. um Repräsentationen des Untersuchungsbereichs nach Applikation einer dritten Menge an Kontrastmittel handeln.

Die beiden Repräsentation O3 und F3 umfassen die gleichen Informationen über den Untersuchungsbereich, nur in einer anderen Darstellung.

Für den Menschen sind die Repräsentationen O1, O2 und O3 des Untersuchungsbereichs im Ortsraum die geläufigen Repräsentationen; der Mensch kann solche Ortsraumdarstellungen unmittelbar erfassen. Die Repräsentationen O1, O2 und O3 zeigen, welchen Einfluss unterschiedliche Mengen an Kontrastmittel auf das Aussehen des Untersuchungsbereichs in einer MR-Untersuchung haben können. Im vorliegenden Fall steigt die Menge an Kontrastmittel von O1 über O2 nach O3 an. Dieselbe Information ist auch in den Repräsentationen F1, F2 und F3 enthalten; sie ist jedoch für den Menschen aus den Darstellungen F1, F2 und F3 schwerer erfassbar.

Es ist auch denkbar, eine andere Transformation als die Fourier-Transformation zu verwenden, um Ortsraum-Repräsentationen in Frequenzraum-Repräsentationen umzuwandeln. Die drei Haupteigenschaften, die eine solche Transformation erfüllen muss, sind:
a) Existenz einer eindeutigen Rücktransformation (eindeutiger Zusammenhang zwischen Ortsraumdarstellung und Frequenzraumdarstellung)
b) Lokalität der Kontrastinformation
c) Robustheit gegenüber mangelhafter Bildregistrierung

Details zur Transformation von einer Darstellung in eine andere sind in einer Vielzahl von Fachbüchern und Publikationen beschrieben (siehe z.B.: W. Burger, M.J. Burge: Digital Image Processing: An Algorithmic Introduction Using Java, Texts in Computer Science, 2. Ausgabe, Springer- Verlag, 2016, ISBN: 9781447166849; W. Birkfellner: Applied Medical Image Processing, Second Edition: A Basic Course, Verlag Taylor & Francis, 2014, ISBN: 9781466555570; R. Bracewell: Fourier Analysis and Imaging, Verlag Springer Science & Business Media, 2004, ISBN: 9780306481871).

Damit das erfindungsgemäße Modell des maschinellen Lernens (Vorhersagemodell) die hier beschriebenen Vorhersagen machen kann, muss es vorab entsprechend konfiguriert (trainiert) werden.

Dabei bedeutet der Begriff "Vorhersage", dass mindestens eine Repräsentation eines Untersuchungsbereichs, die den Untersuchungsbereich nach einer Applikation einer spezifischen Menge an Kontrastmittel im Frequenzraum repräsentiert, auf Basis von mindestens zwei Repräsentationen des Untersuchungsbereichs im Frequenzraum berechnet wird, wobei die mindestens zwei Repräsentationen den Untersuchungsbereich nach Applikation unterschiedlicher Mengen an Kontrastmittel und/oder nach Applikation unterschiedlicher Kontrastmittel repräsentieren.

Mit anderen Worten: es werden mindestens eine erste Repräsentation eines Untersuchungsbereichs eines Untersuchungsobjekts und mindestens eine zweite Repräsentation des Untersuchungsbereichs des Untersuchungsobjekts verwendet, um mindestens eine dritte Repräsentation des Untersuchungsbereichs des Untersuchungsobjekts vorherzusagen. Bei allen Repräsentationen, der mindestens einen ersten, der mindestens einen zweiten und der mindestens einen dritten Repräsentation, handelt es sich um Repräsentationen des Untersuchungsbereichs im Frequenzraum.

Die mindestens eine erste Repräsentation repräsentiert den Untersuchungsbereich nach Applikation einer ersten Menge eines ersten Kontrastmittels, wobei diese erste Menge auch Null (keine Applikation eines Kontrastmittels) sein kann.

Die mindestens eine zweite Repräsentation repräsentiert den Untersuchungsbereich nach Applikation einer zweiten Menge des ersten Kontrastmittels oder einer zweiten Menge eines zweiten Kontrastmittels. Wird zur Erzeugung der zweiten Repräsentation das erste Kontrastmittel verwendet, ist die zweite Menge üblicherweise ungleich der ersten Menge. Wird ein zweites Kontrastmittel verwendet, kann die zweite Menge gleich oder ungleich der ersten Menge sein.

Die mindestens eine dritte Repräsentation repräsentiert den Untersuchungsbereich nach Applikation einer dritten Menge des ersten Kontrastmittels oder einer dritten Menge des zweiten Kontrastmittels oder einer dritten Menge eines dritten Kontrastmittels. Wird zur Erzeugung der ersten Repräsentation, der zweiten Repräsentation und der dritten Repräsentation das gleiche Kontrastmittel verwendet, so ist die dritte Menge üblicherweise ungleich der zweiten Menge und ungleich der ersten Menge; für die erste Menge M1, die zweite Menge M2 und die dritte Menge M3 gilt vorzugsweise: die erste Menge M1 ist größer oder gleich Null, die zweite Menge M2 ist größer als die erste Menge M1 und die dritte Menge M3 ist größer als die zweite Menge M2 (0 ≤ M1 < M2 < M3).

Wird bei der Erzeugung der dritten Repräsentation ein anderes Kontrastmittel verwendet als bei der Erzeugung der ersten und/oder der zweiten Repräsentation, kann die Menge des anderen Kontrastmittels gleich oder ungleich der Menge(n) des/der Kontrastmittel(s) zur Erzeugung der ersten und/oder zweiten Repräsentation sein.

Das Vorhersagemodell kann also trainiert werden, den Einfluss unterschiedlicher Mengen an Kontrastmittel auf Repräsentationen des Untersuchungsbereichs im Frequenzraum zu lernen, es kann aber auch trainiert werden, den Einfluss unterschiedlicher Kontrastmittel auf Repräsentationen des Untersuchungsbereichs im Frequenzraum zu lernen.

Das Vorhersagemodell wird vorzugsweise mit Hilfe eines selbstlernenden Algorithmus in einem überwachten maschinellen Lernen erstellt (konfiguriert, trainiert). Zum Lernen werden Trainingsdaten verwendet. Diese Trainingsdaten umfassen, von einer Vielzahl an Untersuchungsobjekten, für jedes Untersuchungsobjekt eine Mehrzahl von Repräsentationen eines Untersuchungsbereichs. Der Untersuchungsbereich ist für alle Untersuchungsobjekte üblicherweise der gleiche (z.B. ein Teil eines menschlichen Körpers oder ein Organ oder ein Teil eines Organs). Die Repräsentationen des Trainingsdatensatzes werden in dieser Beschreibung auch als Referenz-Repräsentationen bezeichnet.

Für jedes Untersuchungsobjekt umfassen die Trainingsdaten i) mindestens eine erste Referenz-Repräsentation des Untersuchungsbereichs im Frequenzraum, die den Untersuchungsbereich ohne Kontrastmittel oder nach Applikation einer ersten Menge an Kontrastmittel repräsentiert, ii) mindestens eine zweite Referenz-Repräsentationen des Untersuchungsbereichs im Frequenzraum, die den Untersuchungsbereich nach Applikation einer zweiten Menge an Kontrastmittel repräsentiert, und iii) mindestens eine dritte Referenz-Repräsentationen des Untersuchungsbereichs im Frequenzraum, die den Untersuchungsbereich nach Applikation einer dritten Menge an Kontrastmittel repräsentiert. Dabei gilt, wie oben beschrieben: die erste, zweite und dritte Menge an Kontrastmittel und/oder die jeweils verwendeten Kontrastmittel unterscheiden sich voneinander.

Das Vorhersagemodell wird trainiert, für jedes Untersuchungsobjekt die mindestens eine dritte Referenz-Repräsentation auf Basis der mindestens einen ersten Referenz-Repräsentation und der mindestens einen zweiten Referenz-Repräsentation vorherzusagen (zu berechnen).

Der selbstlernende Algorithmus erzeugt beim maschinellen Lernen ein statistisches Modell, das auf den Trainingsdaten beruht. Das heißt, es werden nicht einfach die Beispiele auswendig gelernt, sondern der Algorithmus "erkennt" Muster und Gesetzmäßigkeiten in den Trainingsdaten. So kann das Vorhersagemodell auch unbekannte Daten beurteilen. Validierungsdaten können verwendet werden, um die Güte der Beurteilung unbekannter Daten zu prüfen.

Das Trainieren des Vorhersagemodells kann mittels überwachten Lernens (engl.: *supervised learning*) erfolgen, d.h. dem Algorithmus werden nacheinander Tripel von Datensätzen (erste, zweite und dritte Repräsentationen) präsentiert. Der Algorithmus lernt dann eine Beziehung zwischen diesen Datensätzen.

Selbstlernende Systeme, die mittels überwachten Lernens trainiert werden, sind vielfältig im Stand der Technik beschrieben (siehe z.B. C. Perez: Machine Learning Techniques: Supervised Learning and Classification, Amazon Digital Services LLC - Kdp Print Us, 2019, ISBN 1096996545, 9781096996545).

Vorzugsweise ist das Vorhersagemodell ein künstliches neuronales Netz oder umfasst ein solches.

Ein künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen von ersten und zweiten Repräsentationen. Die Ausgangsneuronen dienen dazu, für mindestens eine erste Repräsentation und mindestens eine zweite Repräsentation, mindestens eine dritte Repräsentation auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN).

Ein Convolutional Neural Network ist in der Lage, Eingabedaten in Form einer Matrix zu verarbeiten. Ein CNN besteht im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabedaten auf gegebene Ausgabedaten angestrebt. Die Qualität der Abbildung wird durch eine Fehlerfunktion (engl.: *loss function*) beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Beziehung zwischen der mindestens einen ersten Repräsentationen und der mindestens einen zweiten Repräsentation auf der einen Seite und der mindestens einen dritten Repräsentation auf der anderen Seite. Diese Informationen können verwendet werden, um mindestens eine dritte Repräsentation auf Basis mindestens einer ersten und mindestens einer zweiten Repräsentation vorherzusagen.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte Daten anwenden lässt.

Weitere Details zum Aufsetzen und Trainieren von künstlichen neuronalen Netzen sind dem Stand der Technik zu entnehmen (siehe z.B.: S. Khan et al.: A Guide to Convolutional Neural Networks for Computer Vision, Morgan & Claypool Publishers 2018, ISBN 1681730227, 9781681730226, WO2018/183044A1, WO2018/200493, WO2019/074938A1, WO2019/204406A1, WO2019/241659A1).

Vorzugsweise ist das Vorhersagemodell ein *Generative Adversarial Network* (GAN) (siehe z.B.: http://3dgan.csail.mit.edu/).

Neben den Repräsentationen können zum Trainieren, Validieren und Vorhersagen auch weitere Informationen zum Untersuchungsobjekt, zum Untersuchungsbereich, zu Untersuchungsbedingungen und/oder zu den radiologischen Untersuchungsmethoden verwendet werden.

Beispiele für Informationen zum Untersuchungsobjekt sind: Geschlecht, Alter, Gewicht, Größe, Anamnese, Art und Dauer und Menge bereits eingenommener Medikamente, Blutdruck, zentralvenöser Druck, Atemfrequenz, Serum Albumin, Total Bilirubin, Blutzucker, Eisengehalt, Atemkapazität und dergleichen. Diese Informationen zum Untersuchungsobjekt können z.B. auch aus einer Datenbank bzw. einer elektronischen Patientenakte ausgelesen werden.

Beispiele für Informationen zum Untersuchungsbereich sind: Vorerkrankungen, Operationen, Teilresektion, Lebertransplantation, Eisenleber, Fettleber und dergleichen.

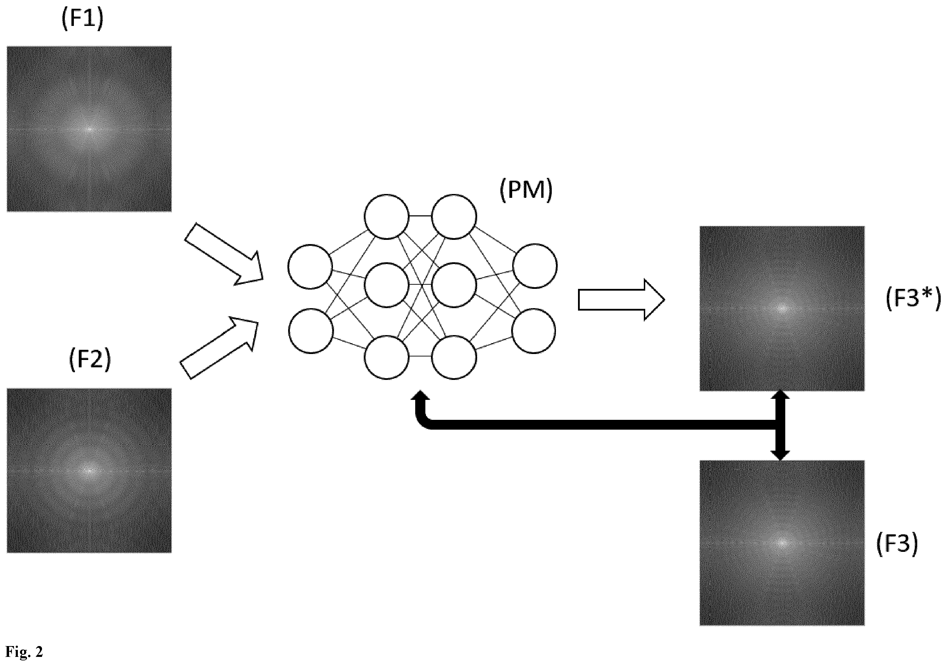

Fig. 2 zeigt schematisch und beispielhaft, wie die in Fig. 1 erzeugten Repräsentationen (F1), (F2) und (F3) des Untersuchungsbereichs im Frequenzraum zum Trainieren eines Vorhersagemodells (PM) genutzt werden können. Die Repräsentationen (F1), (F2) und (F3) bilden einen Satz von Trainingsdaten eines Untersuchungsobjekts. Das Trainieren erfolgt mit einer Vielzahl von Trainingsdatensätzen einer Vielzahl von Untersuchungsobjekten.

Bei den Repräsentationen (F1) und (F2) handelt es sich um eine erste und eine zweite Repräsentation eines Untersuchungsbereichs im Frequenzraum, wobei die Repräsentationen den Untersuchungsbereich nach der Applikation unterschiedlicher Mengen an Kontrastmittel repräsentieren (wobei es auch sein kann, dass in einem Fall kein Kontrastmittel appliziert worden ist).

Bei der Repräsentation (F3) handelt es sich um eine dritte Repräsentation des Untersuchungsbereichs im Frequenzraum, die den Untersuchungsbereich nach der Applikation einer dritten Menge an Kontrastmittel repräsentiert.

Das Vorhersagemodell wird in Fig. 2 trainiert, aus den Repräsentationen (F1) und (F2) des Untersuchungsbereichs im Frequenzraum, die Repräsentation (F3) des Untersuchungsbereichs im Frequenzraum vorherzusagen.

Die Repräsentationen (F1) und (F2) werden in das Vorhersagemodell (PM) eingegeben und das Vorhersagemodell errechnet aus den Repräsentationen (F1) und (F2) eine Repräsentation (F3*). Das Sternchen (*) signalisiert, dass es sich bei der Repräsentation (F3*) um eine vorhergesagte Repräsentation handelt. Die errechnete Repräsentation (F3*) wird mit der Repräsentation (F3) verglichen. Die Abweichungen zwischen der errechneten Repräsentation (F3*) und der gemessenen Repräsentation (F3) können in einem Backpropagation-Verfahren genutzt werden, das Vorhersagemodell zu trainieren, die Abweichungen auf ein definiertes Minimum zu reduzieren. Ist das Vorhersagemodell anhand einer Vielzahl von Trainingsdatensätzen einer Vielzahl an Untersuchungsobjekten trainiert worden und hat die Vorhersage eine definierte Genauigkeit erreicht, kann das trainierte Vorhersagemodell zur Vorhersage verwendet werden.

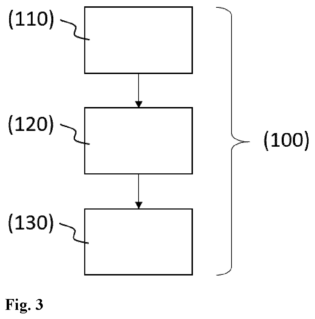

Fig. 3 zeigt beispielhaft und schematisch in Form eines Ablaufschemas eine bevorzugte Ausführungsform des erfindungsgemäßen Verfahrens zum Trainieren eines Modells des maschinellen Lernens.

Das Verfahren (100) umfasst die folgenden Schritte:
(110) Empfangen eines Trainingsdatensatzes, wobei der Trainingsdatensatz für jedes Untersuchungsobjekt einer Vielzahl von Untersuchungsobjekten i) eine erste Referenz-Repräsentation eines Untersuchungsbereichs des Untersuchungsobjekts im Frequenzraum, ii) eine zweite Referenz-Repräsentation des Untersuchungsbereichs des Untersuchungsobjekts im Frequenzraum und iii) eine dritte Referenz-Repräsentation des Untersuchungsbereichs des Untersuchungsobjekts im Frequenzraum umfasst, wobei die erste Referenz-Repräsentation den Untersuchungsbereich nach Applikation einer ersten Menge an Kontrastmittel repräsentiert, wobei die erste Menge auch Null sein kann, wobei die zweite Referenz-Repräsentation den Untersuchungsbereich nach einer Applikation einer zweiten Menge an Kontrastmittel repräsentiert, wobei die dritte Referenz-Repräsentation den Untersuchungsbereich nach Applikation einer dritten Menge an Kontrastmittel repräsentiert,
(120) für jedes Untersuchungsobjekt: Zuführen zumindest eines Teils der ersten Referenz-Repräsentation und zumindest eines Teils der zweiten Referenz-Repräsentation dem Modell des maschinellen Lernens, wobei das Modell des maschinellen Lernens trainiert wird, eine Repräsentation des Untersuchungsbereichs im Frequenzraum nach Applikation der dritten Menge an Kontrastmittel auf Basis der ersten Referenz-Repräsentation und der zweiten Referenz-Repräsentation zu erzeugen, wobei das Trainieren das Minimieren einer Fehlerfunktion umfasst, wobei die Fehlerfunktion Abweichungen der erzeugten Repräsentation des Untersuchungsbereichs von der dritten Referenz-Repräsentation quantifiziert,
(130) Ausgeben und/oder Speichern des trainierten Modells und/oder Zuführen des trainierten Modells einem Verfahren zur Vorhersage einer Repräsentation des Untersuchungsbereichs eines neuen Untersuchungsobjekts.

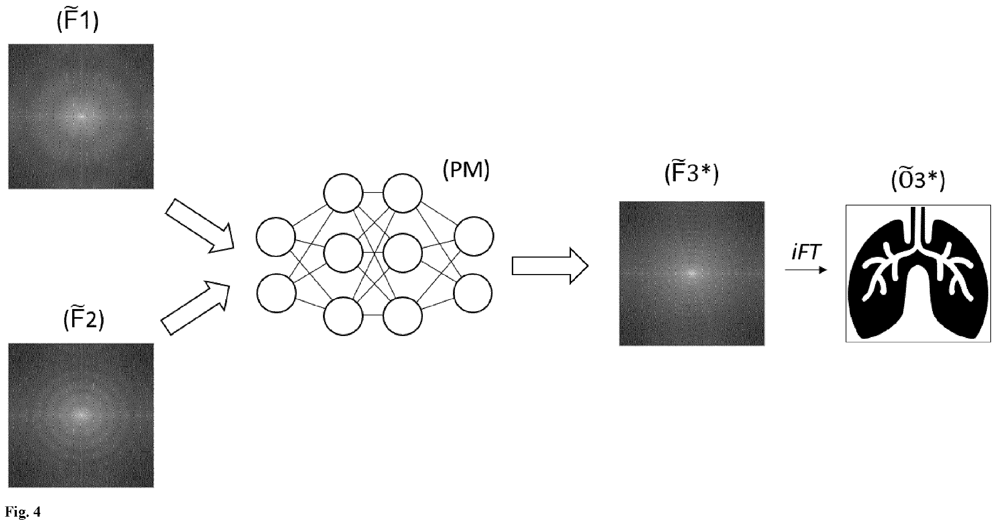

Die Verwendung des trainierten Vorhersagemodells zur Vorhersage ist beispielhaft und schematisch in Fig. 4 dargestellt. Fig. 4 zeigt das in Fig. 2 trainierte Vorhersagemodell (PM). Das Vorhersagemodell wird genutzt, um auf Basis mindestens einer ersten Repräsentation des Untersuchungsbereichs eines Untersuchungsobjekts im Frequenzraum und mindestens einer zweiten Repräsentation des Untersuchungsbereichs des Untersuchungsobjekts im Frequenzraum, mindestens eine dritte Repräsentation des Untersuchungsbereichs des Untersuchungsobjekts im Frequenzraum vorherzusagen, wobei die Repräsentationen den Untersuchungsbereich nach der Applikation unterschiedlicher Mengen an Kontrastmittel repräsentieren.

Im vorliegenden Beispiel wird eine erste Repräsentation (F̃1) und eine zweite Repräsentation (F̃2) des Untersuchungsbereichs im Frequenzraum in das Vorhersagemodell eingegeben und das Vorhersagemodell erzeugt (berechnet) eine dritte Repräsentation (F̃3*). Die Tilde (~) signalisiert, dass es sich bei den Repräsentationen um Repräsentationen von einem neuen Untersuchungsobjekt handelt, von dem üblicherweise keine Repräsentationen vorliegen, die in dem Trainingsverfahren für das Training des Vorhersagemodells verwendet worden sind. Das Sternchen (*) signalisiert, dass es sich bei der Repräsentation (F̃3*) um eine vorhergesagte Repräsentation handelt. Die Repräsentation (F̃3*) des Untersuchungsbereichs im Frequenzraum kann z.B. mittels inverser Fourier-Transformation *iFT* in eine Repräsentation (Õ3*) des Untersuchungsbereichs im Ortsraum transformiert werden.

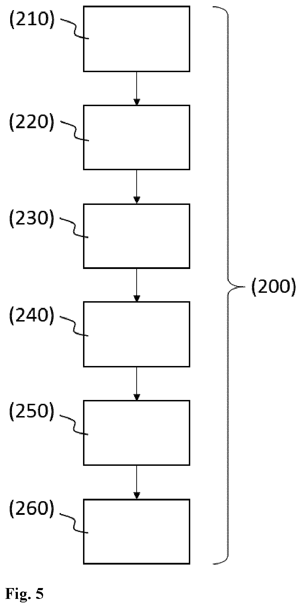

Fig. 5 zeigt beispielhaft und schematisch in Form eines Ablaufschemas eine bevorzugte Ausführungsform des erfindungsgemäßen Verfahrens zur Vorhersage einer Repräsentation eines Untersuchungsbereichs eines Untersuchungsobjekts.

Das Verfahren (200) umfasst die folgenden Schritte:
(210) Empfangen einer ersten Repräsentation eines Untersuchungsbereich eines Untersuchungsobjekts im Frequenzraum, wobei die erste Repräsentation den Untersuchungsbereich ohne Kontrastmittel oder nach Applikation einer ersten Menge an Kontrastmittel repräsentiert,
(220) Empfangen einer zweiten Repräsentation des Untersuchungsbereichs des Untersuchungsobjekts im Frequenzraum, wobei die zweite Repräsentation den Untersuchungsbereich nach einer Applikation einer zweiten Menge an Kontrastmittel repräsentiert,
(230) Zuführen der ersten Repräsentation und der zweiten Repräsentation einem Modell des maschinellen Lernens,
(240) Empfangen, von dem Modell des maschinellen Lernens, einer dritten Repräsentation des Untersuchungsbereichs im Frequenzraum, wobei die dritte Repräsentation den Untersuchungsbereich nach Applikation einer dritten Menge an Kontrastmittel repräsentiert,
(250) Erzeugen einer Repräsentation des Untersuchungsbereichs in einer Ortsraumdarstellung auf Basis der dritten Repräsentation,
(260) Ausgeben und/oder Speichern der Repräsentation des Untersuchungsbereichs in der Ortsraumdarstellung.

Wie bereits beschrieben handelt es sich bei den Repräsentationen des Untersuchungsbereichs, die zum Trainieren, Validieren und zur Vorhersage verwendet werden, um Repräsentationen des Untersuchungsbereichs im Frequenzraum (auch als Ortsfrequenzraum oder Fourier-Raum oder Frequenzdomäne oder Fourier-Darstellung bezeichnet).

In der Magnetresonanztomografie fallen die Rohdaten aufgrund des oben beschriebenen Messverfahrens üblicherweise als so genannte k-Raum-Daten an. Bei diesen k-Raum-Daten handelt es sich um eine Darstellung eines Untersuchungsbereichs in einem Frequenzraum; d.h. solche k-Raum-Daten können zum Trainieren, Validieren und zur Vorhersage verwendet werden. Falls Repräsentationen im Ortsraum vorliegen, können solche Repräsentationen im Ortsraum beispielsweise durch Fourier-Transformation in eine Repräsentation im Frequenzraum umgewandelt (transformiert) werden; umgekehrt gilt: Repräsentationen im Frequenzraum können z.B. durch inverse Fourier-Transformation in eine Repräsentation im Ortsraum umgewandelt (transformiert) werden.

Liegt also eine radiologische Aufnahme eines Untersuchungsbereichs in Form eines zweidimensionalen Bildes im Ortsraum vor, so kann diese Repräsentation des Untersuchungsbereichs durch eine 2D-Fourier-Transformation in eine zweidimensionale Repräsentation des Untersuchungsbereichs im Frequenzraum überführt werden.

Ein dreidimensionales Bild (Volumendarstellung) eines Untersuchungsbereichs kann als ein Stapel von zweidimensionalen Bildern behandelt werden. Denkbar ist ferner, dass das dreidimensionale Bild mittels 3D-Fouriertransformation in eine dreidimensionale Repräsentation des Untersuchungsbereichs im Frequenzraum umgewandelt wird.

Die erfindungsgemäße Verwendung von Repräsentationen des Untersuchungsbereichs im Frequenzraum hat verschiedene Vorteile gegenüber der Verwendung von Repräsentationen des Untersuchungsbereichs im Ortsraum.

Beispielsweise ist die Co-Registrierung der einzelnen Repräsentationen im Frequenzraum weniger kritisch als im Ortsraum. Die "Co-Registrierung" (im Stand der Technik auch "Bildregistrierung" genannt) ist ein wichtiger Prozess in der digitalen Bildverarbeitung und dient dazu, zwei oder mehrere Bilder derselben Szene, oder zumindest ähnlicher Szenen, bestmöglich in Übereinstimmung miteinander zu bringen. Dabei wird eines der Bilder als Referenzbild festgelegt, die anderen werden Objektbilder genannt. Um diese optimal an das Referenzbild anzupassen, wird eine ausgleichende Transformation berechnet. Die zu registrierenden Bilder unterscheiden sich voneinander, weil sie von unterschiedlichen Positionen, zu unterschiedlichen Zeitpunkten und/oder mit unterschiedlichen Sensoren aufgenommen wurden.

Im Fall der vorliegenden Erfindung wurden die einzelnen Repräsentationen zu unterschiedlichen Zeitpunkten erzeugt; zum anderen unterscheiden sie sich hinsichtlich des Gehalts an Kontrastmittel im Untersuchungsbereich und/oder hinsichtlich des verwendeten Kontrastmittels.

Die Verwendung von Repräsentationen des Untersuchungsbereichs im Frequenzraum hat nun gegenüber der Verwendung von Repräsentationen des Untersuchungsbereichs im Ortsraum den Vorteil, dass die Trainings-, Validierungs- und Vorhersageverfahren toleranter gegenüber Fehlern bei der Bildregistrierung sind. Mit anderen Worten: falls Repräsentationen im Frequenzraum nicht genau überlagert werden, hat dies einen geringeren Einfluss, als wenn Repräsentationen im Ortsraum nicht Pixel/Voxel-genau überlagert werden. Dies folgt aus den Eigenschaften der Fourier-Transformation: wie bereits beschrieben wird die Kontrastinformation von Fourier-transformierten Bildern stets in der Nähe des Ursprungs des Fourier-Raumes abgebildet. Drehungen oder Rotationen im Bildraum (Ortsraum) führen dazu, dass Bildinformationen (z.B. eine sichtbare Struktur) nach der Transformation in einem anderen Bereich des Bildes lokalisiert sind. Im Fourier-Raum ändern diese Transformationen jedoch nicht den Bereich, in dem die für die vorliegenden Erfindung relevante Kontrastinformation kodiert ist.

Ein weiterer Vorteil der Verwendung von Repräsentation im Frequenzraum besteht darin, dass Kontrastinformationen, auf die es beim Training und bei der Vorhersage ankommt, von Detailinformationen (Feinstrukturen) getrennt werden können. Es ist also möglich, sich bei dem Training auf die Informationen zu konzentrieren, die das Vorhersagemodell lernen soll, und sich bei der Vorhersage ebenfalls auf die Informationen zu konzentrieren, die das Vorhersagemodell vorhersagen soll: Kontrastinformationen.

Während Kontrastinformationen in einer Repräsentation eines Untersuchungsbereichs im Ortsraum üblicherweise über die gesamte Repräsentation verteilt sind (jedes Pixel/Voxel trägt Informationen zum Kontrast in sich), sind die Kontrastinformationen in einer Repräsentation eines Untersuchungsbereichs im Frequenzraum im und um das Zentrum des Frequenzraums herum kodiert. Mit anderen Worten: die niedrigen Frequenzen in einer Repräsentation im Frequenzraum sind für den Kontrast verantwortlich, während die hohen Frequenzen Informationen zu Feinstrukturen enthalten.

Es ist damit möglich, die Kontrastinformationen zu separieren, das Training und die Vorhersage auf die Kontrastinformationen zu beschränken und Informationen zu den Feinstrukturen nach dem Training / nach der Vorhersage wieder zuzuführen.

Dazu kann in den Repräsentationen des Untersuchungsbereichs, die zum Trainieren, Validieren und zur Vorhersage verwendet werden, ein Bereich spezifiziert werden.

Das Spezifizieren des Bereichs kann beispielsweise dadurch erfolgen, dass ein Nutzer des erfindungsgemäßen Computersystems einen oder mehrere Parameter in das erfindungsgemäße Computersystem eingibt und/oder aus einer Liste auswählt, die die Form und/oder Größe des Bereichs definieren. Denkbar ist aber auch, dass das Spezifizieren automatisch beispielsweise durch das erfindungsgemäße Computersystem erfolgt, das entsprechend konfiguriert wurde, einen vordefinierten Bereich in den Repräsentationen des Untersuchungsbereichs auszuwählen.

Der spezifizierte Bereich ist üblicherweise kleiner als der von der jeweiligen Repräsentation ausgefüllte Frequenzraum; umfasst aber in jedem Fall das Zentrum des Frequenzraums.

Ein Bereich der Repräsentation, der das Zentrum des Frequenzraums (auch Ursprung oder Nullpunkt genannt) umfasst, beinhaltet die für das erfindungsgemäße Verfahren relevante Kontrastinformation. Ist der spezifizierte Bereich kleiner als der von der jeweiligen Repräsentation ausgefüllte Frequenzraum, so ergibt sich für das Training, die Validierung und die nachfolgende Vorhersage ein geringerer Rechenaufwand. Durch die Wahl der Größe des Bereichs kann also direkt Einfluss auf den Rechenaufwand genommen werden.

Es ist prinzipiell auch möglich, einen Bereich zu spezifizieren, der dem gesamten Frequenzraum entspricht, der von der jeweiligen Repräsentation ausgefüllt wird; in einem solchen Fall findet keine Reduktion auf einen Teilbereich des Frequenzraums statt und der Rechenaufwand ist maximal.

Der Nutzer des erfindungsgemäßen Computersystems kann also durch Spezifizieren eines Bereichs um das Zentrum des Frequenzraums herum selbst entscheiden, ob er ein Training, eine Validierung und eine Vorhersage auf Basis der vollständigen Repräsentationen des Untersuchungsbereichs im Frequenzraum wünscht (was einen maximalen Rechenaufwand bedeutet) oder ob er den Rechenaufwand reduzieren möchte, indem er einen Bereich spezifiziert, der kleiner ist, als der von den Repräsentationen ausgefüllte Frequenzraum. Dabei kann er durch die Größe des spezifizierten Bereichs direkten Einfluss auf den erforderlichen Rechenaufwand ausüben.

Der spezifizierte Bereich hat üblicherweise dieselbe Dimension wie der Frequenzraum: im Fall einer 2D-Repräsentation in einem 2D-Frequenzraum ist der spezifizierte Bereich üblicherweise eine Fläche; im Fall einer 3D-Repräsentation in einem 3D-Frequenzraum ist der spezifizierte Bereich üblicherweise ein Volumen.

Der spezifizierte Bereich kann prinzipiell eine beliebige Form aufweisen; er kann also beispielsweise rund und/oder eckig, konkav und/oder konvex sein. Vorzugsweise ist der Bereich quader- oder würfelförmig im Fall eines 3D-Frequenzraums in einem kartesischen Koordinatensystem und rechteckig oder quadratisch im Fall eines 2D-Frequenzraums in einem kartesischen Koordinatensystem. Es ist aber auch denkbar, dass der spezifizierte Bereich kugel- oder kreisförmig ist oder eine andere Form aufweist.

Vorzugsweise fällt der geometrische Schwerpunkt des spezifizierten Bereichs mit dem Zentrum des Frequenzraums zusammen.

Die Repräsentationen, die zum Trainieren, Validieren und zur Vorhersage verwendet werden, werden auf den spezifizierten Bereich reduziert. Dabei bedeutet der Begriff "reduzieren", dass alle Teile einer Repräsentation, die nicht in dem spezifizierten Bereich liegen, weggeschnitten (verworfen) oder durch eine Maske bedeckt werden. Bei der Maskierung werden diejenigen Bereiche, die außerhalb des spezifizierten Bereichs liegen, mit einer Maske bedeckt, so dass nur der spezifizierte Bereich unbedeckt bleibt; bei der Bedeckung mit einer Maske können zum Beispiel die Farbwerte der entsprechenden Pixel/Voxel auf Null (schwarz) gesetzt werden.

Die so erhaltenen Repräsentationen werden in dieser Beschreibung auch als reduzierte Repräsentationen bezeichnet.

Die reduzierten Repräsentationen können zum Trainieren, Validieren und Vorhersagen verwendet werden.

Soll also für ein Untersuchungsobjekt eine dritte Repräsentation eines Untersuchungsbereichs auf Basis einer ersten und einer zweiten Repräsentation vorhergesagt werden, so können die reduzierte erste Repräsentation und die reduzierte zweite Repräsentation dem Vorhersagemodell zugeführt werden, und das Vorhersagemodell erzeugt dann eine reduzierte dritte Repräsentation. In einem weiteren Schritt können die Detailinformationen, die beim Reduzieren weggeschnitten oder bedeckt wurden, der vorhergesagten Repräsentation wieder zugeführt werden. Es können also beispielsweise die Teile der ersten Repräsentation, die außerhalb des spezifizierten Bereichs liegen, der reduzierten dritten Repräsentation zugefügt werden. Denkbar ist auch, dass die Teile der zweiten Repräsentation, die außerhalb des spezifizierten Bereichs liegen, der reduzierten dritten Repräsentation zugefügt werden. Denkbar ist auch, dass sowohl Teile der ersten Repräsentation, die außerhalb des spezifizierten Bereichs liegen, also auch Teile der zweiten Repräsentation, die außerhalb des spezifizierten Bereichs liegen, der reduzierten dritten Repräsentation zugeführt werden. Mit anderen Worten: die dritte Repräsentation wird durch Teile der ersten und/oder der zweiten Repräsentation ergänzt, die beim Reduzieren weggeschnitten/mit einer Maske bedeckt worden sind. Das Ergebnis ist eine ergänzte dritte Repräsentation. Aus der ergänzten dritten Repräsentation kann dann durch eine Transformation (zum Beispiel eine inverse Fourier-Transformation) eine Ortsraumdarstellung gewonnen werden.

Die sich dabei ergebenden Signalstärken für verschiedene Ortskoordinaten können in einem weiteren Schritt in Grauwerte oder Farbwerte umgewandelt werden, um ein digitales Bild in einem gängigen Bildformat (z.B. DICOM) vorliegen zu haben.

Die Repräsentation des Untersuchungsbereichs in der Ortsraumdarstellung kann auf einem Bildschirm angezeigt, auf einem Drucker ausgegeben und/oder in einem Datenspeicher gespeichert werden.

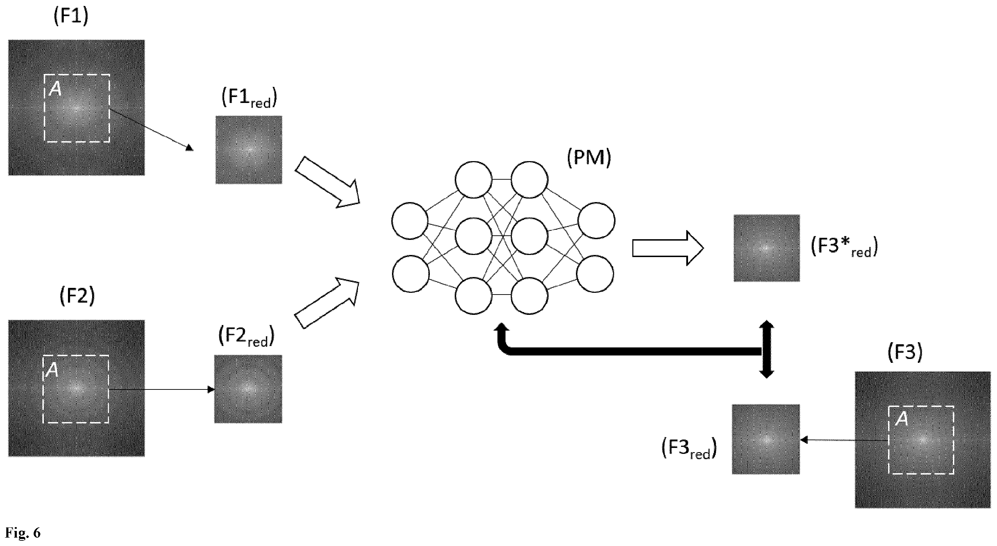

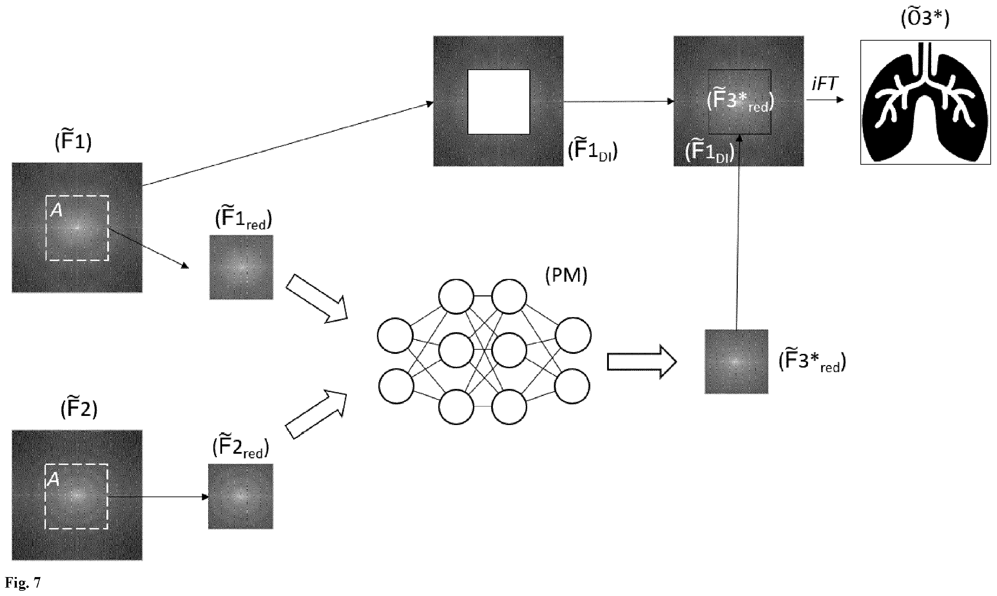

Das beschriebene Vorgehen wird nachfolgend beispielhaft anhand von Fig. 6 und Fig. 7 näher erläutert.

Fig. 6 zeigt beispielhaft und schematisch einen Schritt beim Trainieren eines Vorhersagemodells gemäß einer bevorzugten Ausführungsform der vorliegenden Erfindung.

Es werden, wie im Fall von Fig. 2, eine erste Repräsentation (F1), eine zweite Repräsentation (F2) und eine dritte Repräsentation (F3) eines Untersuchungsbereichs eines Untersuchungsobjekts im Frequenzraum empfangen. In den Repräsentationen (F1), (F2) und (F3) wird jeweils ein Bereich *A* derselben Größe und Form spezifiziert. Der Bereich *A* umfasst jeweils das Zentrum des Frequenzraumes und hat im vorliegenden Fall eine quadratische Form, wobei der geometrische Schwerpunkt des Quadrats mit dem Zentrum des Frequenzraums zusammenfällt. Die Repräsentationen (F1), (F2) und (F3) werden auf den jeweils spezifizierten Bereich *A* reduziert: es ergeben sich drei reduzierte Repräsentationen (F1_{red}), (F2_{red}) und (F3_{red}). Die reduzierten Repräsentationen werden für das Training verwendet. Das Vorhersagemodell wird trainiert, aus den reduzierten Repräsentationen (F1_{red}) und (F2_{red}) die reduzierte Repräsentation (F3_{red}) vorherzusagen. Die reduzierten Repräsentationen (F1_{red}) und (F2_{red}) werden dem Vorhersagemodell (PM) zugeführt und das Vorhersagemodell errechnet eine reduzierte Repräsentation (F3*_{red}), die der reduzierten Repräsentation (F3_{red}) möglichst nahe kommen soll.

Fig. 7 zeigt beispielhaft und schematisch wie das in Fig. 6 trainierte Vorhersagemodell für die Vorhersage verwendet werden kann.

Im vorliegenden Beispiel werden eine erste Repräsentation (F̃1) und eine zweite Repräsentation (F̃2) des Untersuchungsbereichs im Frequenzraum empfangen und jeweils auf einen spezifizierten Bereich *A* reduziert. Es ergeben sich zwei reduzierte Repräsentationen (F̃1_{red}) und (F̃2_{red}). Die reduzierte erste Repräsentation (F̃1_{red}) und die reduzierte zweite Repräsentation (F̃2_{red}) werden dem trainierten Vorhersagemodell (PM) zugeführt. Das trainierte Vorhersagemodell (PM) berechnet aus den reduzierten Repräsentationen (F̃1_{red}) und (F̃2_{red}) eine reduzierte dritte Repräsentation (F̃3_{red}*). Die reduzierte dritte Repräsentation (F̃3_{red}*) wird in einem weiteren Schritt um denjenigen Bereich der empfangenen ersten Repräsentation (F̃1) ergänzt, der beim Reduzieren der empfangenen ersten Repräsentation (F̃1) verworfen wurde. Mit anderen Worten; es werden diejenigen Teile der empfangenen ersten Repräsentation (F̃1) zu der reduzierten dritten Repräsentation (F̃3_{red}*) ergänzt, die außerhalb (nicht innerhalb) des spezifizierten Bereichs liegen. Wie beschrieben können anstelle von oder in Ergänzung zu Teilen der empfangenen ersten Repräsentation (F̃1) auch Teile der empfangenen zweiten Repräsentation (F̃2) zu der reduzierten dritten Repräsentation (F̃3_{red}*) ergänzt werden.

Aus der ergänzten Repräsentation (F̃3_{red}*) + (F̃1_{DI}) kann durch inverse Fourier-Transformation eine Repräsentation des Untersuchungsbereichs im Ortsraum (Õ3*) erzeugt werden.

Es sei angemerkt, dass auch andere Methoden zur Transformation einer Frequenzraum-Darstellung in eine Ortsraum-Darstellung verwendet werden können, wie z.B. iterative Rekonstruktionsverfahren.

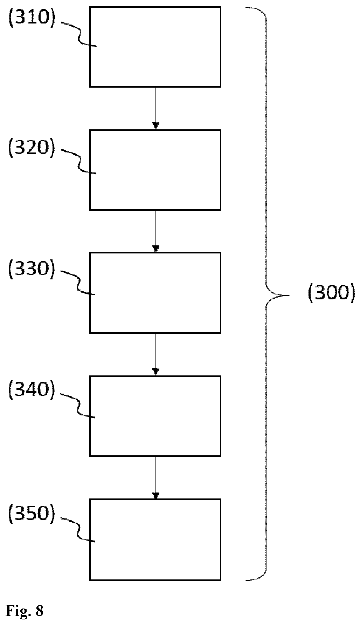

Fig. 8 zeigt schematisch in Form eines Ablaufdiagramms eine bevorzugte Ausführungsform für das erfindungsgemäße Verfahren zum Trainieren eines Modells des maschinellen Lernens.

Das Verfahren (300) umfasst die Schritte:
(310) Empfangen eines Trainingsdatensatzes, wobei der Trainingsdatensatz für jedes Untersuchungsobjekt einer Vielzahl von Untersuchungsobjekten i) eine erste Referenz-Repräsentation eines Untersuchungsbereichs des Untersuchungsobjekts im Frequenzraum, ii) eine zweite Referenz-Repräsentation des Untersuchungsbereichs des Untersuchungsobjekts im Frequenzraum und iii) eine dritte Referenz-Repräsentation des Untersuchungsbereichs des Untersuchungsobjekts im Frequenzraum umfasst, wobei die erste Referenz-Repräsentation den Untersuchungsbereich nach Applikation einer ersten Menge an Kontrastmittel repräsentiert, wobei die erste Menge auch Null sein kann, wobei die zweite Referenz-Repräsentation den Untersuchungsbereich nach einer Applikation einer zweiten Menge an Kontrastmittel repräsentiert, wobei die dritte Referenz-Repräsentation den Untersuchungsbereich nach Applikation einer dritten Menge an Kontrastmittel repräsentiert,
(320) Spezifizieren eines Bereichs in den Referenz-Repräsentationen, wobei der spezifizierte Bereich das Zentrum des Frequenzraums umfasst,
(330) Reduzieren der Repräsentationen auf den spezifizierten Bereich, wobei für jedes Untersuchungsobjekt eine reduzierte erste Referenz-Repräsentation, eine reduzierte zweite Referenz-Repräsentation und eine reduzierte dritte Referenz-Repräsentation erhalten werden,
(340) für jedes Untersuchungsobjekt: Zuführen der reduzierten ersten Referenz-Repräsentation und der reduzierten zweiten Referenz-Repräsentation dem Modell des maschinellen Lernens, wobei das Modell des maschinellen Lernens trainiert wird, eine reduzierte Repräsentation des Untersuchungsbereichs im Frequenzraum nach Applikation der dritten Menge an Kontrastmittel auf Basis der reduzierten ersten Referenz-Repräsentation und der reduzierten zweiten Referenz-Repräsentation zu generieren, wobei das Trainieren das Minimieren einer Fehlerfunktion umfasst, wobei die Fehlerfunktion Abweichungen der generierten reduzierten Repräsentation des Untersuchungsbereichs von der reduzierten dritten Referenz-Repräsentation quantifiziert,
(350) Ausgeben und/oder Speichern des trainierten Modells und/oder Zuführen des trainierten Modells einem Verfahren zur Vorhersage einer Repräsentation des Untersuchungsbereichs eines neuen Untersuchungsobjekts.

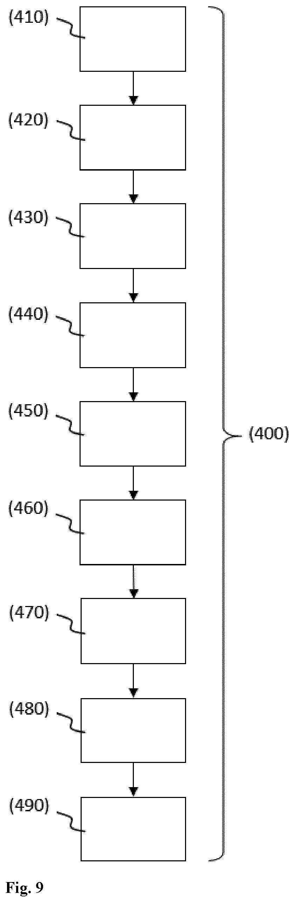

Fig. 9 zeigt schematisch in Form eines Ablaufdiagramms eine bevorzugte Ausführungsform für das erfindungsgemäße Verfahren zum Vorhersagen einer Repräsentation eines Untersuchungsbereichs eines Untersuchungsobjekts.

Das Verfahren (400) umfasst die Schritte:
(410) Empfangen einer ersten Repräsentation eines Untersuchungsbereich eines Untersuchungsobjekts im Frequenzraum, wobei die erste Repräsentation den Untersuchungsbereich ohne Kontrastmittel oder nach Applikation einer ersten Menge an Kontrastmittel repräsentiert,
(420) Empfangen einer zweiten Repräsentation des Untersuchungsbereichs des Untersuchungsobjekts im Frequenzraum, wobei die zweite Repräsentation den Untersuchungsbereich nach einer Applikation einer zweiten Menge an Kontrastmittel repräsentiert,
(430) Spezifizieren eines Bereichs in der ersten Repräsentation und in der zweiten Repräsentation, wobei der spezifizierte Bereich das Zentrum des Frequenzraums umfasst,
(440) Reduzieren der ersten Repräsentation und der zweiten Repräsentation auf den spezifizierten Bereich, wobei eine reduzierte erste Repräsentation und eine reduzierte zweite Repräsentation erhalten werden,
(450) Zuführen der reduzierten ersten Repräsentation und der reduzierten zweiten Repräsentation einem Modell des maschinellen Lernens,
(460) Empfangen, von dem Modell des maschinellen Lernens, einer dritten Repräsentation des Untersuchungsbereichs im Frequenzraum, wobei die dritte Repräsentation den Untersuchungsbereich nach Applikation einer dritten Menge an Kontrastmittel repräsentiert,
(470) Ergänzen der dritten Repräsentation um diejenigen Teile der empfangenen ersten und/oder der empfangenen zweiten Repräsentation, die nicht in dem spezifizierten Bereich liegen,
(480) Transformieren der nach Ergänzen der dritten Repräsentation erhaltenen ergänzten dritten Repräsentation in eine Repräsentation des Untersuchungsbereichs in einer Ortsraumdarstellung,
(490) Ausgeben und/oder Speichern der Repräsentation des Untersuchungsbereichs in der Ortsraumdarstellung.

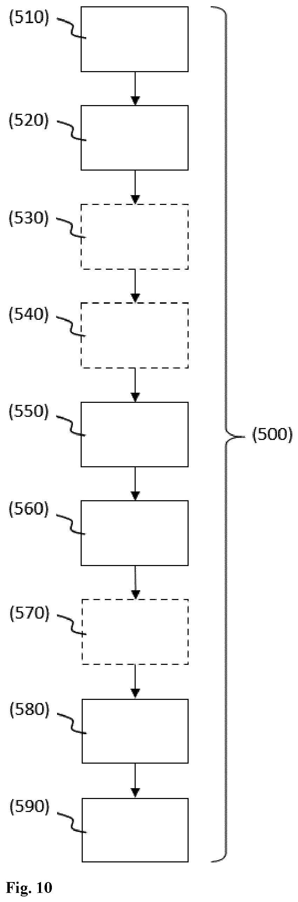

Fig. 10 zeigt schematisch in Form eines Ablaufdiagramms eine weitere bevorzugte Ausführungsform für das erfindungsgemäße Verfahren zum Vorhersagen einer Repräsentation eines Untersuchungsbereichs eines Untersuchungsobjekts.

Das Verfahren (500) umfasst die Schritte:
(510) Bereitstellen eines trainierten Modells des maschinellen Lernens, wobei das Modell nach dem oben beschriebenen Verfahren (100) oder (300) trainiert worden ist, den Einfluss der Menge an Kontrastmittel auf Repräsentationen des Untersuchungsbereichs im Frequenzraum zu lernen,
(520) Empfangen einer ersten Repräsentation des Untersuchungsbereich des Untersuchungsobjekts im Frequenzraum, wobei die erste Repräsentation den Untersuchungsbereich nach Applikation einer ersten Menge eines Kontrastmittels repräsentiert, wobei die erste Menge auch Null sein kann, und Empfangen einer zweiten Repräsentation des Untersuchungsbereichs des Untersuchungsobjekts im Frequenzraum, wobei die zweite Repräsentation den Untersuchungsbereich nach einer Applikation einer zweiten Menge des Kontrastmittels repräsentiert,
(530) optional: Spezifizieren eines Bereichs in der ersten Repräsentation und in der zweiten Repräsentation, wobei der spezifizierte Bereich das Zentrum des Frequenzraums umfasst,
(540) optional: Reduzieren der ersten Repräsentation und der zweiten Repräsentation auf den spezifizierten Bereich, wobei eine reduzierte erste Repräsentation und eine reduzierte zweite Repräsentation erhalten werden,
(550) Zuführen
   - der empfangenen ersten Repräsentation und der empfangenen zweiten Repräsentation
   - oder, wenn vorhanden, der reduzierten ersten Repräsentation und der reduzierten zweiten Repräsentation
   dem Modell des maschinellen Lernens,
(560) Empfangen, von dem Modell des maschinellen Lernens, einer dritten Repräsentation des Untersuchungsbereichs im Frequenzraum, wobei die dritte Repräsentation den Untersuchungsbereich nach Applikation einer dritten Menge des Kontrastmittels repräsentiert,
(570) optional: Ergänzen der dritten Repräsentation durch diejenigen Teile der empfangenen ersten und/oder der empfangenen zweiten Repräsentation, die nicht in dem spezifizierten Bereich liegen, wobei eine ergänzte dritte Repräsentation erhalten wird,
(580) Erzeugen einer Repräsentation des Untersuchungsbereichs in einer Ortsraumdarstellung aus
   - der dritten Repräsentation,
   - oder, wenn vorhanden, der ergänzten dritten Repräsentation,
(590) Ausgeben der Repräsentation des Untersuchungsbereichs in der Ortsraumdarstellung.

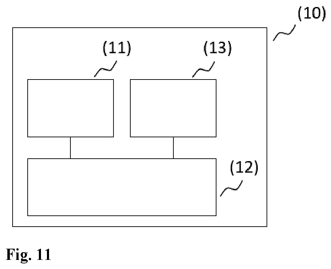

Figur 11 zeigt schematisch und beispielhaft eine Ausführungsform des erfindungsgemäßen Computersystems. Das Computersystem (10) umfasst eine Empfangseinheit (11), eine Steuer- und Recheneinheit (12) und eine Ausgabeeinheit (13).

Ein "Computersystem" ist ein System zur elektronischen Datenverarbeitung, das mittels programmierbarer Rechenvorschriften Daten verarbeitet. Ein solches System umfasst üblicherweise eine Steuer- und Recheneinheit, oftmals auch als "Computer" bezeichnet, diejenige Einheit, die einen Prozessor zur Durchführung logischer Operationen und einen Arbeitsspeicher zum Laden eines Computerprogramms umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Joystick, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem (zum Bespiel zur Steuerung durch einen Nutzer) erfolgen über Eingabemittel wie beispielsweise eine Tastatur, eine Maus, ein Mikrofon, ein berührungsempfindliches Display und/oder dergleichen. Ausgaben erfolgen über die Ausgabeeinheit (13), die insbesondere ein Monitor (Bildschirm), ein Drucker und/oder ein Datenspeicher sein kann.

Das erfindungsgemäße Computersystem (10) ist konfiguriert, aus mindestens zwei Repräsentationen eines Untersuchungsbereich im Frequenzraum, die den Untersuchungsbereich nach Applikation unterschiedlicher Mengen an Kontrastmittel repräsentieren, eine Repräsentation des Untersuchungsbereichs vorherzusagen, die den Untersuchungsbereich nach der Applikation einer spezifischen Menge an Kontrastmittel zeigt, ohne dass diese spezifische Menge tatsächlich verabreicht werden muss.

Die Steuer- und Recheneinheit (12) dient der Steuerung der Empfangseinheit (11) und der Ausgabeeinheit (13), der Koordinierung der Daten- und Signalflüsse zwischen den verschiedenen Einheiten, der Prozessierung von Repräsentationen des Untersuchungsbereichs und der Erzeugung von künstlichen radiologischen Aufnahmen. Es ist denkbar, dass mehrere Steuer- und Recheneinheiten vorhanden sind

Die Empfangseinheit (11) dient zum Empfang von Repräsentationen eines Untersuchungsbereichs. Die Repräsentationen können beispielsweise von einem Magnetresonanztomographen übermittelt werden oder von einem Computertomographen übermittelt werden oder aus einem Datenspeicher ausgelesen werden. Der Magnetresonanztomograph oder der Computertomograph können ein Bestandteil des erfindungsgemäßen Computersystems sein. Denkbar ist aber auch, dass das erfindungsgemäße Computersystem ein Bestandteil eines Magnetresonanztomographen oder eines Computertomographen ist. Die Übermittlung von Repräsentationen kann über eine Netzwerkverbindung oder eine direkte Verbindung erfolgen. Die Übermittlung von Repräsentationen kann über eine Funkverbindung (WLAN, Bluetooth, Mobilfunk und/oder dergleichen) und/oder kabelgebunden erfolgen. Es ist denkbar, dass mehrere Empfangseinheiten vorhanden sind. Auch der Datenspeicher kann ein Bestandteil des erfindungsgemäßen Computersystems sein oder mit diesem z.B. über ein Netzwerk verbunden sein. Es ist denkbar, dass mehrere Datenspeicher vorhanden sind.

Von der Empfangseinheit werden die Repräsentationen und ggf. weitere Daten (wie beispielsweise Informationen zum Untersuchungsobjekt, Aufnahmeparameter und/oder dergleichen) empfangen und an die Steuer- und Recheneinheit übermittelt.

Die Steuer- und Recheneinheit ist konfiguriert, anhand der empfangenen Daten künstliche radiologische Aufnahmen zu erzeugen.

Über die Ausgabeeinheit (13) können die künstlichen radiologischen Aufnahmen angezeigt werden (zum Beispiel auf einem Monitor), ausgegeben werden (z.B. über einen Drucker) und/oder in einem Datenspeicher gespeichert werden. Es ist denkbar, dass mehrere Ausgabeeinheiten vorhanden sind.

Wie bereits beschrieben kann die Erfindung eingesetzt werden, um die Menge an Kontrastmittel in einer radiologischen Untersuchung zu reduzieren. Das Vorhersagemodell kann trainiert werden, auf Basis einer ersten Repräsentation eines Untersuchungsbereichs eines Untersuchungsobjekts, die den Untersuchungsbereich ohne Kontrastmittel oder nach Applikation einer ersten Menge eines Kontrastmittel repräsentiert, und auf Basis einer zweiten Repräsentation des Untersuchungsbereichs des Untersuchungsobjekts, die den Untersuchungsbereich nach einer Applikation einer zweiten Menge des Kontrastmittels repräsentiert, eine dritte Repräsentation des Untersuchungsbereichs des Untersuchungsbereichs des Untersuchungsobjekts vorherzusagen, wobei die dritte Repräsentation den Untersuchungsbereich nach Applikation einer dritten Menge des Kontrastmittels repräsentiert, wobei die dritte Menge größer als die erste Menge und die zweite Menge ist. Es muss also lediglich eine erste Menge (die auch Null sein kann) und eine zweite Menge des Kontrastmittels appliziert werden, um radiologische Aufnahmen zu erzeugen, die so aussehen, als wäre eine größere dritte Menge an Kontrastmittel appliziert worden.

Die Erfindung kann eingesetzt werden, um künstliche radiologische Aufnahmen zu erzeugen, die einen Untersuchungsbereich eines Untersuchungsobjekts nach Applikation eines Kontrastmittels zeigen, obwohl ein anderes Kontrastmittel appliziert worden ist.

Die Erfindung kann eingesetzt werden, um auf Basis von radiologischen Aufnahme einer radiologischen Untersuchung (z.B. einer Magnetresonanztomographie) eine künstliche radiologische Aufnahme zu erzeugen, die das Ergebnis einer anderen radiologischen Untersuchung zeigt (z.B. das Ergebnis einer Computertomographie) zeigt.

Weitere Anwendungen sind denkbar.

## Patentansprüche

1. Computer-implementiertes Verfahren umfassend die Schritte:
- Empfangen einer ersten Repräsentation (F̃1) eines Untersuchungsbereich eines Untersuchungsobjekts, wobei die erste Repräsentation (F̃1) den Untersuchungsbereich ohne Kontrastmittel oder nach Applikation einer ersten Menge an Kontrastmittel repräsentiert,
- Empfangen einer zweiten Repräsentation (F̃2) des Untersuchungsbereichs des Untersuchungsobjekts, wobei die zweite Repräsentation (F̃2) den Untersuchungsbereich nach einer Applikation einer zweiten Menge an Kontrastmittel repräsentiert,
- Zuführen zumindest eines Teils der ersten Repräsentation (F̃1) und zumindest eines Teils der zweiten Repräsentation (F̃2) einem Modell (PM) des maschinellen Lernens,
- Empfangen, von dem Modell (PM) des maschinellen Lernens, einer dritten Repräsentation (F̃3*) des Untersuchungsbereichs, wobei die dritte Repräsentation (F̃3*) den Untersuchungsbereich nach Applikation einer dritten Menge an Kontrastmittel repräsentiert,
- Ausgeben und/oder Speichern einer Repräsentation (Õ3*) des Untersuchungsbereichs in einer Ortsraumdarstellung,
**dadurch gekennzeichnet, dass** die erste Repräsentation (F̃1), die zweite Repräsentation (F̃2) und die dritte Repräsentation (F̃3*) den Untersuchungsbereich des Untersuchungsobjekts im Frequenzraum repräsentieren,
wobei das Verfahren ferner umfasst:
- Erzeugen der Repräsentation (Õ3*) des Untersuchungsbereichs in der Ortsraumdarstellung auf Basis der dritten Repräsentation (F̃3*).

2. Computer-implementiertes Verfahren gemäß Anspruch 1,
wobei der Schritt Zuführen zumindest eines Teils der ersten Repräsentation (F̃1) und zumindest eines Teils der zweiten Repräsentation (F̃2) einem Modell des maschinellen Lernens (PM) umfasst:
- Spezifizieren eines Bereichs (*A*) in der empfangenen ersten Repräsentation (F̃1) und in der empfangenen zweiten Repräsentation (F̃2), wobei der spezifizierte Bereich (*A*) das Zentrum des Frequenzraums umfasst,
- Reduzieren der ersten Repräsentation (F̃1) und der zweiten Repräsentation (F̃2) auf den spezifizierten Bereich (*A*), wobei eine reduzierte erste Repräsentation (F̃1_{red}) und eine reduzierte zweite Repräsentation (F̃2_{red}) erhalten werden,
- Zuführen der reduzierten ersten Repräsentation (F̃1_{red}) und der reduzierten zweiten Repräsentation (F2_{red}) dem Modell (PM) des maschinellen Lernens.

3. Computer-implementiertes Verfahren gemäß Anspruch 2,
wobei der Schritt Erzeugen einer Repräsentation (Õ3*) des Untersuchungsbereichs in einer Ortsraumdarstellung auf Basis der dritten Repräsentation (F̃3*) umfasst:
- Ergänzen der dritten Repräsentation (F̃3*red) um diejenigen Teile (F̃1_{DI}) der empfangenen ersten Repräsentation (F̃1) und/oder der empfangenen zweiten Repräsentation (F̃2), die nicht in dem spezifizierten Bereich (*A*) liegen,
- Transformieren der nach Ergänzen der dritten Repräsentation (F̃3*_{red}) erhaltenen ergänzten dritten Repräsentation in die Repräsentation (Õ3*) des Untersuchungsbereichs in der Ortsraumdarstellung.

4. Computer-implementiertes Verfahren gemäß einem der Ansprüche 1 bis 3, wobei die erste Repräsentation (F̃1) den Untersuchungsbereich ohne Kontrastmittel oder nach Applikation der ersten Menge eines Kontrastmittels repräsentiert, wobei die zweite Repräsentation (F̃2) den Untersuchungsbereich nach einer Applikation einer zweiten Menge des Kontrastmittels repräsentiert, wobei die zweite Menge ungleich der ersten Menge ist, wobei die dritte Repräsentation (F̃3*) den Untersuchungsbereich nach Applikation einer dritten Menge des Kontrastmittels repräsentiert, wobei die dritte Menge ungleich der ersten Menge und/oder ungleich der zweiten Menge ist.

5. Computer-implementiertes Verfahren gemäß einem der Ansprüche 1 bis 4, wobei die erste Menge an Kontrastmittel größer oder gleich Null ist, wobei die zweite Menge an Kontrastmittel größer als die erste Menge an Kontrastmittel ist und wobei die dritte Menge an Kontrastmittel größer als die zweite Menge an Kontrastmittel ist.

6. Computer-implementiertes Verfahren gemäß einem der Ansprüche 1 bis 5, wobei die erste Repräsentation (F̃1) den Untersuchungsbereich ohne Kontrastmittel oder nach Applikation einer ersten Menge eines ersten Kontrastmittels repräsentiert, wobei die zweite Repräsentation (F̃2) den Untersuchungsbereich nach einer Applikation einer zweiten Menge eines zweiten Kontrastmittels repräsentiert, wobei die dritte Repräsentation (F̃3*) den Untersuchungsbereich nach Applikation einer dritten Menge eines dritten Kontrastmittels repräsentiert, wobei das dritte Kontrastmittel ungleich dem ersten Kontrastmittel und/oder ungleich dem zweiten Kontrastmittel ist oder das zweite Kontrastmittel ungleich dem ersten Kontrastmittel ist.

7. Computer-implementiertes Verfahren gemäß einem der Ansprüche 1 bis 6, wobei die erste Repräsentation (F̃1) und die zweite Repräsentation (F̃2) das Ergebnis einer radiologischen Untersuchung sind.

8. Computer-implementiertes Verfahren gemäß Anspruch 7, wobei es sich bei der radiologischen Untersuchung um eine magnetresonanztomografische Untersuchung, um eine computertomografische Untersuchung oder um eine Ultraschalluntersuchung handelt.

9. Computer-implementiertes Verfahren gemäß einem der Ansprüche 1 bis 8, wobei es sich bei der ersten Repräsentation (F̃1) und der zweiten Repräsentation (F̃2) um k-Raum-Daten einer magnetresonanztomografischen Untersuchung handelt.

10. Computer-implementiertes Verfahren gemäß einem der Ansprüche 1 bis 9, wobei es sich bei der ersten Repräsentation (F̃1) und der zweiten Repräsentation (F̃2) um Fourier-transformierte Ortsraumdarstellungen handelt.

11. Computer-implementiertes Verfahren gemäß einem der Ansprüche 1 bis 7, ferner umfassend die Schritte:
- Empfangen einer ersten Ortsraum-Repräsentation (O1) des Untersuchungsbereich des Untersuchungsobjekts, wobei die erste Ortsraum-Repräsentation (O1) den Untersuchungsbereich ohne Kontrastmittel oder nach Applikation einer ersten Menge an Kontrastmittel repräsentiert,
- Empfangen einer zweiten Ortsraum-Repräsentation (O2) des Untersuchungsbereichs des Untersuchungsobjekts, wobei die zweite Ortsraum-Repräsentation (O2) den Untersuchungsbereich nach einer Applikation einer zweiten Menge an Kontrastmittel repräsentiert,
- Erzeugen der ersten Repräsentation (F̃1) des Untersuchungsbereichs des Untersuchungsobjekts im Frequenzraum aus der ersten Ortsraum-Repräsentation (O1), vorzugsweise mittels Fourier-Transformation (*FT*)*.*
- Erzeugen der zweiten Repräsentation (F̃2) des Untersuchungsbereichs des Untersuchungsobjekts im Frequenzraum aus der zweiten Ortsraum-Repräsentation, (O2) vorzugsweise mittels Fourier-Transformation *(FT).*

12. Computer-implementiertes Verfahren gemäß einem der Ansprüche 1 bis 11, ferner umfassend den Schritt: Trainieren des Modells des maschinellen Lernens, wobei das Trainieren die folgenden Unterschritte umfasst:
- Empfangen eines Trainingsdatensatzes, wobei der Trainingsdatensatz für eine Vielzahl von Untersuchungsobjekten jeweils i) eine erste Referenz-Repräsentation (F1) eines Untersuchungsbereichs des Untersuchungsobjekts im Frequenzraum, ii) eine zweite Referenz-Repräsentation (F2) des Untersuchungsbereichs des Untersuchungsobjekts im Frequenzraum und iii) eine dritte Referenz-Repräsentation (F3) des Untersuchungsbereichs des Untersuchungsobjekts im Frequenzraum umfasst, wobei die erste Referenz-Repräsentation (F1) den Untersuchungsbereich nach Applikation einer ersten Menge eines Kontrastmittels repräsentiert, wobei die erste Menge auch Null sein kann, wobei die zweite Referenz-Repräsentation (F2) den Untersuchungsbereich nach einer Applikation einer zweiten Menge des Kontrastmittels repräsentiert, wobei die dritte Referenz-Repräsentation (F3) den Untersuchungsbereich nach Applikation einer dritten Menge des Kontrastmittels repräsentiert,
- Spezifizieren eines Bereichs (*A*) in den Referenz-Repräsentationen, wobei der spezifizierte Bereich (*A*) das Zentrum des Frequenzraums umfasst,
- Reduzieren der Repräsentationen (F1, F2, F3) auf den spezifizierten Bereich (*A*), wobei für jedes Untersuchungsobjekt eine reduzierte erste Referenz-Repräsentation (F1_{red}), eine reduzierte zweite Referenz-Repräsentation (F2_{red}) und eine reduzierte dritte Referenz-Repräsentation (F3_{red}) erhalten werden,
- für jedes Untersuchungsobjekt: Zuführen der reduzierten ersten Referenz-Repräsentation (F1_{red}) und der reduzierten zweiten Referenz-Repräsentation (F2_{red}) dem Modell (PM) des maschinellen Lernens, wobei das Modell (PM) des maschinellen Lernens trainiert wird, eine reduzierte Repräsentation (F3*_{red}) des Untersuchungsbereichs im Frequenzraum nach Applikation der dritten Menge an Kontrastmittel auf Basis der reduzierten ersten Referenz-Repräsentation (F1_{red}) und der reduzierten zweiten Referenz-Repräsentation (F2_{red}) zu generieren, wobei das Trainieren das Minimieren einer Fehlerfunktion umfasst, wobei die Fehlerfunktion Abweichungen der generierten reduzierten Repräsentation (F3*_{red}) des Untersuchungsbereichs von der reduzierten dritten Referenz-Repräsentation (F3_{red}) quantifiziert.

13. Computersystem (10) umfassend
• eine Empfangseinheit (11),
• eine Steuer- und Recheneinheit (12) und
• eine Ausgabeeinheit (13),
- wobei die Steuer- und Recheneinheit (12) konfiguriert ist, die Empfangseinheit (11) zu veranlassen, mindestens zwei Repräsentationen eines Untersuchungsbereichs eines Untersuchungsobjekts zu empfangen, eine erste Repräsentation (F̃1) und eine zweite Repräsentation (F̃2), wobei die erste Repräsentation (F̃1) den Untersuchungsbereich ohne Kontrastmittel oder nach Applikation einer ersten Menge an Kontrastmittels repräsentiert, wobei die zweite Repräsentation (F̃2) den Untersuchungsbereich nach einer Applikation einer zweiten Menge an Kontrastmittel repräsentiert,
- wobei die Steuer- und Recheneinheit (12) konfiguriert ist, zumindest einen Teil der ersten Repräsentation (F̃1) und zumindest einen Teil der zweiten Repräsentation (F̃2) einem Modell (PM) des maschinellen Lernens zuzuführen,
- wobei die Steuer- und Recheneinheit (12) konfiguriert ist, von dem Modell (PM) des maschinellen Lernens eine dritte Repräsentation (F̃3*) des Untersuchungsbereichs zu empfangen, wobei die dritte Repräsentation (F̃3*) den Untersuchungsbereich nach Applikation einer dritten Menge an Kontrastmittel repräsentiert,
- wobei die Steuer- und Recheneinheit (12) konfiguriert ist, auf Basis der dritten Repräsentation (F̃3*) eine Repräsentation (Õ3*) des Untersuchungsbereichs im Ortsraum zu erzeugen,
- wobei die Steuer- und Recheneinheit (12) konfiguriert ist, die Ausgabeeinheit (13) zu veranlassen, eine Repräsentation (Õ3*) des Untersuchungsbereichs im Ortsraum auszugeben und/oder zu speichern
**dadurch gekennzeichnet, dass** die erste Repräsentation (F̃1), die zweite Repräsentation (F̃2) und die dritte Repräsentation (F̃3*) den Untersuchungsbereich des Untersuchungsobjekts im Frequenzraum repräsentieren,
wobei die Steuer- und Recheneinheit (12) ferner konfiguriert ist, auf Basis der dritten Repräsentation (F̃3*) die Repräsentation (Õ3*) des Untersuchungsbereichs im Ortsraum zu erzeugen.

14. Computerprogramm, das in einen Arbeitsspeicher eines Computersystems (10) geladen werden kann und dort das Computersystem (10) dazu veranlasst, folgende Schritte ausführen:
- Empfangen einer ersten Repräsentation (F̃1) eines Untersuchungsbereich eines Untersuchungsobjekts, wobei die erste Repräsentation (F̃1) den Untersuchungsbereich ohne Kontrastmittel oder nach Applikation einer ersten Menge an Kontrastmittel repräsentiert,
- Empfangen einer zweiten Repräsentation (F̃2) des Untersuchungsbereichs des Untersuchungsobjekts, wobei die zweite Repräsentation (F̃2) den Untersuchungsbereich nach einer Applikation einer zweiten Menge an Kontrastmittel repräsentiert,
- Zuführen zumindest eines Teils der ersten Repräsentation (F̃1) und zumindest eines Teils der zweiten Repräsentation (F̃2) einem Modell (PM) des maschinellen Lernens,
- Empfangen, von dem Modell (PM) des maschinellen Lernens, einer dritten Repräsentation (F3*) des Untersuchungsbereichs, wobei die dritte Repräsentation (F3*) den Untersuchungsbereich nach Applikation einer dritten Menge an Kontrastmittel repräsentiert,
- Ausgeben und/oder Speichern einer Repräsentation (Õ3*) des Untersuchungsbereichs in einer Ortsraumdarstellung
**dadurch gekennzeichnet, dass** die erste Repräsentation (F̃1), die zweite Repräsentation (F̃2) und die dritte Repräsentation (F̃3*) den Untersuchungsbereich des Untersuchungsobjekts im Frequenzraum repräsentieren,
wobei das Computerprogramm das Computersystem (10) ferner veranlasst folgenden Schritt auszuführen:
- Erzeugen der Repräsentation (Õ3*) des Untersuchungsbereichs in der Ortsraumdarstellung auf Basis der dritten Repräsentation (F̃3*).

## Claims

1. Computer-implemented method comprising the steps of:
- receiving a first representation (F̃1) of an examination region of an examination object, the first representation (F̃1) representing the examination region without contrast agent or after administration of a first amount of contrast agent,
- receiving a second representation (F̃2) of the examination region of the examination object, the second representation (F̃2) representing the examination region after an administration of a second amount of contrast agent,
- feeding at least part of the first representation (F̃1) and at least part of the second representation (F̃2) to a machine learning model (PM),
- receiving from the machine learning model (PM) a third representation (F̃3*) of the examination region, the third representation (F̃3*) representing the examination region after administration of a third amount of contrast agent,
- outputting and/or storing a representation (Õ3*) of the examination region in a real-space depiction,
**characterized in that** the first representation (F̃1), the second representation (F̃2) and the third representation (F̃3*) represent the examination region of the examination object in frequency space,
the method further comprising:
- generating the representation (Õ3*) of the examination region in the real-space depiction on the basis of the third representation (F̃3*).

2. Computer-implemented method according to Claim 1, wherein the step of feeding at least part of the first representation (F̃1) and at least part of the second representation (F̃2) to a machine learning model (PM) comprises:
- specifying a region (A) in the received first representation (F̃1) and in the received second representation (F̃2), the specified region (A) comprising the centre of the frequency space,
- reducing the first representation (F̃1) and the second representation (F̃2) to the specified region (A), a reduced first representation (F̃1_{red}) and a reduced second representation (F2_{red}) being obtained,
- feeding the reduced first representation (F̃1_{red}) and the reduced second representation (F2_{red}) to the machine learning model (PM).

3. Computer-implemented method according to Claim 2, wherein the step of generating a representation (Õ3*) of the examination region in a real-space depiction on the basis of the third representation (F̃3*) comprises:
- supplementing the third representation (F̃3*_{red}) by those parts (F̃1_{DI}) of the received first representation (F̃1) and/or the received second representation (F̃2) that do not lie in the specified region (A),
- transforming the supplemented third representation, obtained after the third representation (F̃3*_{red}) has been supplemented, into the representation (Õ3*) of the examination region in the real-space depiction.

4. Computer-implemented method according to any of Claims 1 to 3, wherein the first representation (F̃1) represents the examination region without contrast agent or after administration of the first amount of a contrast agent, wherein the second representation (F̃2) represents the examination region after an administration of a second amount of the contrast agent, the second amount being unequal to the first amount, wherein the third representation (F̃3*) represents the examination region after administration of a third amount of the contrast agent, the third amount being unequal to the first amount and/or unequal to the second amount.

5. Computer-implemented method according to any of Claims 1 to 4, wherein the first amount of contrast agent is greater than or equal to zero, wherein the second amount of contrast agent is greater than the first amount of contrast agent and wherein the third amount of contrast agent is greater than the second amount of contrast agent.

6. Computer-implemented method according to any of Claims 1 to 5, wherein the first representation (F̃1) represents the examination region without contrast agent or after administration of a first amount of a first contrast agent, wherein the second representation (F̃2) represents the examination region after an administration of a second amount of a second contrast agent, wherein the third representation (F̃3*) represents the examination region after administration of a third amount of a third contrast agent, the third contrast agent being different from the first contrast agent and/or different from the second contrast agent or the second contrast agent being different from the first contrast agent.

7. Computer-implemented method according to any of Claims 1 to 6, wherein the first representation (F̃1) and the second representation (F̃2) are the result of a radiological examination.

8. Computer-implemented method according to Claim 7, wherein the radiological examination is a magnetic resonance imaging examination, a computed tomography examination or an ultrasound examination.

9. Computer-implemented method according to any of Claims 1 to 8, wherein the first representation (F̃1) and the second representation (F̃2) are k-space data of a magnetic resonance imaging examination.

10. Computer-implemented method according to any of Claims 1 to 9, wherein the first representation (F̃1) and the second representation (F̃2) are Fourier-transformed real-space depictions.

11. Computer-implemented method according to any of Claims 1 to 7, further comprising the steps of:
- receiving a first real-space representation (O1) of the examination region of the examination object, the first real-space representation (O1) representing the examination region without contrast agent or after administration of a first amount of contrast agent,
- receiving a second real-space representation (O2) of the examination region of the examination object, the second real-space representation (O2) representing the examination region after an administration of a second amount of contrast agent,
- generating the first representation (F̃1) of the examination region of the examination object in frequency space from the first real-space representation (O1), preferably by means of Fourier transform (*FT*).
- generating the second representation (F̃2) of the examination region of the examination object in frequency space from the second real-space representation (O2), preferably by means of Fourier transform (*FT*).

12. Computer-implemented method according to any of Claims 1 to 11, further comprising the step of: training the machine learning model, the training comprising the following sub-steps:
- receiving a training data set, the training data set comprising, for a multiplicity of examination objects, in each case i) a first reference representation (F1) of an examination region of the examination object in frequency space, ii) a second reference representation (F2) of the examination region of the examination object in frequency space and iii) a third reference representation (F3) of the examination region of the examination object in frequency space, the first reference representation (F1) representing the examination region after administration of a first amount of a contrast agent, it also being possible for the first amount to be zero, the second reference representation (F2) representing the examination region after an administration of a second amount of the contrast agent, the third reference representation (F3) representing the examination region after administration of a third amount of the contrast agent,
- specifying a region (A) in the reference representations, the specified region (A) comprising the centre of the frequency space,
- reducing the representations (F1, F2, F3) to the specified region (*A*), a reduced first reference representation (F1_{red}), a reduced second reference representation (F2_{red}) and a reduced third reference representation (F3_{red}) being obtained for each examination object,
- for each examination object: feeding the reduced first reference representation (F1_{red}) and the reduced second reference representation (F2_{red}) to the machine learning model (PM), the machine learning model (PM) being trained to generate a reduced representation (F3*_{red}) of the examination region in frequency space after administration of the third amount of contrast agent on the basis of the reduced first reference representation (F1_{red}) and the reduced second reference representation (F2_{red}), the training comprising the minimization of a loss function, the loss function quantifying deviations of the generated reduced representation (F3*_{red}) of the examination region from the reduced third reference representation (F3_{red}).

13. Computer system (10) comprising
• a receiving unit (11),
• a control and calculation unit (12) and
• an output unit (13),
- wherein the control and calculation unit (12) is configured to cause the receiving unit (11) to receive at least two representations of an examination region of an examination object, a first representation (F̃1) and a second representation (F̃2), the first representation (F̃1) representing the examination region without contrast agent or after administration of a first amount of contrast agent, the second representation (F̃2) representing the examination region after an administration of a second amount of contrast agent,
- wherein the control and calculation unit (12) is configured to feed at least part of the first representation (F̃1) and at least part of the second representation (F̃2) to a machine learning model (PM),
- wherein the control and calculation unit (12) is configured to receive from the machine learning model (PM) a third representation (F̃3*) of the examination region, the third representation (F̃3*) representing the examination region after administration of a third amount of contrast agent,
- wherein the control and calculation unit (12) is configured to generate on the basis of the third representation (F̃3*) a representation (Õ3*) of the examination region in real space,
- wherein the control and calculation unit (12) is configured to cause the output unit (13) to output and/or store a representation (Õ3*) of the examination region in real space,
**characterized in that** the first representation (F̃1), the second representation (F̃2) and the third representation (F̃3*) represent the examination region of the examination object in frequency space,
wherein the control and calculation unit (12) is further configured to generate on the basis of the third representation (F̃3*) the representation (Õ3*) of the examination region in real space.

14. Computer program that can be loaded into a working memory of a computer system (10), where it causes the computer system (10) to execute the following steps:
- receiving a first representation (F̃1) of an examination region of an examination object, the first representation (F̃1) representing the examination region without contrast agent or after administration of a first amount of contrast agent,
- receiving a second representation (F̃2) of the examination region of the examination object, the second representation (F̃2) representing the examination region after an administration of a second amount of contrast agent,
- feeding at least part of the first representation (F̃1) and at least part of the second representation (F̃2) to a machine learning model (PM),
- receiving from the machine learning model (PM) a third representation (F̃3*) of the examination region, the third representation (F̃3*) representing the examination region after administration of a third amount of contrast agent,
- outputting and/or storing a representation (Õ3*) of the examination region in a real-space depiction,
**characterized in that** the first representation (F̃1), the second representation (F̃2) and the third representation (F̃3*) represent the examination region of the examination object in frequency space,
wherein the computer program also causes the computer system (10) to execute the following step:
- generating the representation (Õ3*) of the examination region in the real-space depiction on the basis of the third representation (F̃3*).

## Revendications

1. Procédé mis en œuvre par ordinateur, comprenant les étapes suivantes consistant à :
- recevoir une première représentation (F̃1) d'une zone d'examen d'un objet d'examen, la première représentation (F̃1) représentant la zone d'examen sans agent de contraste ou après application d'une première quantité d'agent de contraste,
- recevoir une deuxième représentation (F̃2) de la zone d'examen de l'objet d'examen, la deuxième représentation (F̃2) représentant la zone d'examen après une application d'une deuxième quantité d'agent de contraste,
- amener au moins une partie de la première représentation (F̃1) et au moins une partie de la deuxième représentation (F̃2) à un modèle (PM) d'apprentissage automatique,
- recevoir, à partir du modèle (PM) d'apprentissage automatique, une troisième représentation (F̃3*) de la zone d'examen, la troisième représentation (F̃3*) représentant la zone d'examen après application d'une troisième quantité d'agent de contraste,
- sortir et/ou enregistrer une représentation (Õ3*) de la zone d'examen dans une visualisation du domaine spatial,
**caractérisé en ce que** la première représentation (F̃1), la deuxième représentation (F̃2) et la troisième représentation (F̃3*) représentent la zone d'examen de l'objet d'examen dans le domaine fréquentiel,
le procédé comprenant en outre les étapes suivantes consistant à :
- produire la représentation (Õ3*) de la zone d'examen dans la visualisation du domaine spatial sur la base de la troisième représentation (F̃3*).

2. Procédé mis en œuvre par ordinateur selon la revendication 1,
dans lequel l'étape consistant à amener au moins une partie de la première représentation (F̃1) et au moins une partie de la deuxième représentation (F̃2) à un modèle d'apprentissage automatique (PM) comprend les étapes suivantes consistant à :
- spécifier une zone (A) dans la première représentation reçue (F̃1) et dans la deuxième représentation reçue (F̃2), la zone spécifiée (A) comprenant le centre du domaine fréquentiel,
- réduire la première représentation (F̃1) et la deuxième représentation (F̃2) à la zone spécifiée (A), une première représentation réduite (F̃1_{red}) et une deuxième représentation réduite (F̃2_{red}) étant ainsi obtenues,
- amener la première représentation réduite (F̃1_{red}) et la deuxième représentation réduite (F2_{red}) au modèle (PM) d'apprentissage automatique.

3. Procédé mis en œuvre par ordinateur selon la revendication 2,
dans lequel l'étape consistant à produire une représentation (Õ3*) de la zone d'examen dans une visualisation du domaine spatial sur la base de la troisième représentation (F̃3*) comprend les étapes suivantes consistant à :
- compléter la troisième représentation (F̃3*_{red}) par les parties (F̃1_{DI}) de la première représentation reçue (F̃1) et/ou de la deuxième représentation reçue (F̃2) qui ne se trouvent pas dans la zone spécifiée (*A*),
- transformer la troisième représentation complétée obtenue après avoir complété la troisième représentation (F̃3*_{red}) en la représentation (Õ3*) de la zone d'examen dans la visualisation du domaine spatial.

4. Procédé mis en œuvre par ordinateur selon l'une quelconque des revendications 1 à 3, dans lequel la première représentation (F̃1) représente la zone d'examen sans agent de contraste ou après application de la première quantité d'un agent de contraste, dans lequel la deuxième représentation (F̃2) représente la zone d'examen après application d'une deuxième quantité de l'agent de contraste, la deuxième quantité étant différente de la première quantité, dans lequel la troisième représentation (F̃3*) représente la zone d'examen après application d'une troisième quantité de l'agent de contraste, la troisième quantité étant différente de la première quantité et/ou différente de la deuxième quantité.

5. Procédé mis en œuvre par ordinateur selon l'une quelconque des revendications 1 à 4, dans lequel la première quantité d'agent de contraste est supérieure ou égale à zéro, dans lequel la deuxième quantité d'agent de contraste est supérieure à la première quantité d'agent de contraste et dans lequel la troisième quantité d'agent de contraste est supérieure à la deuxième quantité d'agent de contraste.

6. Procédé mis en œuvre par ordinateur selon l'une quelconque des revendications 1 à 5, dans lequel la première représentation (F̃1) représente la zone d'examen sans agent de contraste ou après application d'une première quantité d'un premier agent de contraste, dans lequel la deuxième représentation (F̃2) représente la zone d'examen après application d'une deuxième quantité d'un deuxième agent de contraste, dans lequel la troisième représentation (F̃3*) représente la zone d'examen après application d'une troisième quantité d'un troisième agent de contraste, le troisième agent de contraste étant différent du premier agent de contraste et/ou différent du deuxième agent de contraste ou le deuxième agent de contraste étant différent du premier agent de contraste.

7. Procédé mis en œuvre par ordinateur selon l'une quelconque des revendications 1 à 6, dans lequel la première représentation (F̃1) et la deuxième représentation (F̃2) sont le résultat d'un examen radiologique.

8. Procédé mis en œuvre par ordinateur selon la revendication 7, dans lequel l'examen radiologique est un examen d'imagerie par résonance magnétique, un examen par tomographie informatisée ou un examen par ultrasons.

9. Procédé mis en œuvre par ordinateur selon l'une quelconque des revendications 1 à 8, dans lequel la première représentation (F̃1) et la deuxième représentation (F̃2) sont des données d'espace k d'un examen d'imagerie par résonance magnétique.

10. Procédé mis en œuvre par ordinateur selon l'une quelconque des revendications 1 à 9, dans lequel la première représentation (F̃1) et la deuxième représentation (F̃2) sont des visualisations du domaine spatial transformées de Fourier.

11. Procédé mis en œuvre par ordinateur selon l'une quelconque des revendications 1 à 7, comprenant en outre les étapes suivantes consistant à :
- recevoir une première représentation du domaine spatial (O1) de la zone d'examen de l'objet d'examen, la première représentation du domaine spatial (O1) représentant la zone d'examen sans agent de contraste ou après application d'une première quantité d'agent de contraste,
- recevoir une deuxième représentation du domaine spatial (O2) de la zone d'examen de l'objet d'examen, la deuxième représentation du domaine spatial (O2) représentant la zone d'examen après application d'une deuxième quantité d'agent de contraste,
- produire la première représentation (F̃1) de la zone d'examen de l'objet d'examen dans le domaine fréquentiel à partir de la première représentation du domaine spatial (O1), de préférence au moyen d'une transformation de Fourier (*FT*).
- produire la deuxième représentation (F̃2) de la zone d'examen de l'objet d'examen dans le domaine fréquentiel à partir de la deuxième représentation du domaine spatial (O2), de préférence au moyen d'une transformation de Fourier (*FT*).

12. Procédé mis en œuvre par ordinateur selon l'une quelconque des revendications 1 à 11, comprenant en outre l'étape suivante consistant à : former le modèle d'apprentissage automatique, la formation comprenant les sous-étapes suivantes consistant à :
- recevoir un ensemble de données d'apprentissage, l'ensemble de données d'apprentissage comprenant, pour une pluralité d'objets d'examen, respectivement i) une première représentation de référence (F1) d'une zone d'examen de l'objet d'examen dans le domaine fréquentiel, ii) une deuxième représentation de référence (F2) de la zone d'examen de l'objet d'examen dans le domaine fréquentiel et iii) une troisième représentation de référence (F3) de la zone d'examen de l'objet d'examen dans le domaine fréquentiel, la première représentation de référence (F1) représentant la zone d'examen après application d'une première quantité d'un agent de contraste, la première quantité pouvant également être nulle, la deuxième représentation de référence (F2) représentant la zone d'examen après application d'une deuxième quantité de l'agent de contraste, la troisième représentation de référence (F3) représentant la zone d'examen après application d'une troisième quantité de l'agent de contraste,
- spécifier une zone (*A*) dans les représentations de référence, la zone spécifiée (A) comprenant le centre du domaine fréquentiel,
- réduire les représentations (F1, F2, F3) à la zone spécifiée (*A*), une première représentation de référence réduite (F1_{red}), une deuxième représentation de référence réduite (F2_{red}) et une troisième représentation de référence réduite (F3_{red}) étant obtenues pour chaque objet d'examen,
- pour chaque objet d'examen : amener la première représentation de référence réduite (F1_{red}) et la deuxième représentation de référence réduite (F2_{red}) au modèle (PM) d'apprentissage automatique, le modèle (PM) d'apprentissage automatique étant formé pour générer une représentation réduite (F3*_{red}) de la région d'examen dans le domaine fréquentiel après application de la troisième quantité d'agent de contraste sur la base de la première représentation de référence réduite (F1_{red}) et de la deuxième représentation de référence réduite (F2_{red}), l'apprentissage comprenant la minimisation d'une fonction d'erreur, la fonction d'erreur quantifiant des écarts entre la représentation réduite générée (F3*_{red}) de la zone d'examen et la troisième représentation de référence réduite (F3_{red}).

13. Système informatique (10) comprenant
• une unité de réception (11),
• une unité de commande et de calcul (12), et
• une unité de sortie (13),
- dans lequel l'unité de commande et de calcul (12) est configurée pour amener l'unité de réception (11) à recevoir au moins deux représentations d'une zone d'examen d'un objet d'examen, une première représentation (F̃1) et une deuxième représentation (F̃2), la première représentation (F̃1) représentant la zone d'examen sans agent de contraste ou après application d'une première quantité d'agent de contraste, la deuxième représentation (F̃2) représentant la zone d'examen après application d'une deuxième quantité d'agent de contraste,
- dans lequel l'unité de commande et de calcul (12) est configurée pour amener au moins une partie de la première représentation (F̃1) et au moins une partie de la deuxième représentation (F̃2) à un modèle (PM) d'apprentissage automatique,
- dans lequel l'unité de commande et de calcul (12) est configurée pour recevoir du modèle (PM) d'apprentissage automatique une troisième représentation (F̃3*) de la zone d'examen, la troisième représentation (F̃3*) représentant la zone d'examen après application d'une troisième quantité d'agent de contraste,
- dans lequel l'unité de commande et de calcul (12) est configurée pour produire sur la base de la troisième représentation (F̃3*) une représentation (Õ3*) de la zone d'examen dans le domaine spatial,
- dans lequel l'unité de commande et de calcul (12) est configurée pour amener l'unité de sortie (13) à sortir et/ou à enregistrer une représentation (Õ3*) de la zone d'examen dans le domaine spatial
**caractérisé en ce que** la première représentation (F̃1), la deuxième représentation (F̃2) et la troisième représentation (F̃3*) représentent la zone d'examen de l'objet d'examen dans le domaine fréquentiel,
dans lequel l'unité de commande et de calcul (12) est en outre configurée pour produire, sur la base de la troisième représentation (F̃3*), la représentation (Õ3*) de la zone d'examen dans le domaine spatial.

14. Programme informatique, qui peut être chargé dans une mémoire de travail d'un système informatique (10) et qui y amène le système informatique (10) à exécuter les étapes suivantes consistant à :
- recevoir une première représentation (F̃1) d'une zone d'examen d'un objet d'examen, la première représentation (F̃1) représentant la zone d'examen sans agent de contraste ou après application d'une première quantité d'agent de contraste,
- recevoir une deuxième représentation (F̃2) de la zone d'examen de l'objet d'examen, la deuxième représentation (F̃2) représentant la zone d'examen après une application d'une deuxième quantité d'agent de contraste,
- amener au moins une partie de la première représentation (F̃1) et au moins une partie de la deuxième représentation (F̃2) à un modèle (PM) d'apprentissage automatique,
- recevoir, à partir du modèle (PM) d'apprentissage automatique, une troisième représentation (F̃3*) de la zone d'examen, la troisième représentation (F̃3*) représentant la zone d'examen après application d'une troisième quantité d'agent de contraste,
- sortir et/ou enregistrer une représentation (Õ3*) de la zone d'examen dans une visualisation du domaine spatial,
**caractérisé en ce que** la première représentation (F̃1), la deuxième représentation (F̃2) et la troisième représentation (F̃3*) représentent la zone d'examen de l'objet d'examen dans le domaine fréquentiel,
dans lequel le programme informatique amène en outre le système informatique (10) à exécuter l'étape suivante consistant à :
- produire la représentation (Õ3*) de la zone d'examen dans la visualisation du domaine spatial sur la base de la troisième représentation (F̃3*).
